# EUROPÄISCHE PATENTSCHRIFT

(11) **EP 3 835 402 B1**
(45) Veröffentlichungstag und Bekanntmachung des Hinweises auf die Patenterteilung: **15.10.2025**
(21) Anmeldenummer: 19214525.8
(22) Anmeldetag: 09.12.2019
(51) Int. Cl.: C12M 1/00, C12M 3/00, C12M 1/02, C12M 1/34

(54) **MODULARES PROZESSIERSYSTEM**
MODULAR PROCESSING SYSTEM
SYSTÈME DE TRAITEMENT MODULAIRE

(43) Veröffentlichungstag der Anmeldung: 16.06.2021
(73) Patentinhaber: Sartorius Stedim Biotech GmbH, 37079 Göttingen (DE)
(72) Erfinder: MANZKE, Christian, 37079 Göttingen (DE); BÖTTCHER, Lars, 37079 Göttingen (DE)
(74) Vertreter: Novagraaf International SA

(56) Entgegenhaltungen:
- EP-A2- 0 154 845
- WO-A1-2018/158273
- DE-A1- 102005 008 924
- DE-A1- 3 441 249
- US-A- 5 593 580

## Beschreibung

Die vorliegende Erfindung betrifft ein modulares Prozessiersystem für biopharmazeutische und/oder chemische Prozesse und ein Verfahren zum zentralen Regeln eines modularen Prozessiersystems für biopharmazeutische und/oder chemische Prozesse.

Aus der Biopharmazie sind Prozesse wie beispielsweise Zellabtrennung (beispielsweise durch Tiefenfiltration), Sterilfiltration, Chromatographieschritte, Virusinaktivierung, Virusfiltration und/oder Crossflow-Filtration bekannt. Alle diese Prozesse stellen Grundoperationen dar, die regelmäßig zu unterschiedlichen Gesamtprozessen verschaltet werden. Prozessiersysteme, in denen derartige Gesamtprozesse verschaltet sind, erfordern eine umfassende Überwachung des Fluidstroms bzw. des Fluids, um sicherzustellen, dass das Fluid im Gesamtprozess die erforderlichen Parameter aufweist. Dies benötigt jedoch eine Vielzahl von Messpunkten in dem Prozessiersystem sowie eine aufwendige Verwaltung der umfassenden Überwachungsdaten.

In der Druckschrift WO 2018/158273 A1 ist beispielsweise eine modulare Bioverarbeitungseinheit offenbart, welche umfasst: ein Gehäuse mit einem oder mehreren internen Fluidwegen, wobei das Gehäuse mindestens einen Einlass und mindestens einen Auslass, jeweils in Fluidverbindung mit dem Fluidpfad oder einem oder mehreren der Fluidpfade; ein oder mehrere Sensorelemente, die mit dem oder jedem Pfad operativ verbunden sind; eine oder mehrere Fluidströmungs-induzierende Komponenten, die operativ mit dem oder jedem Fluidpfad verbunden sind; und mehrere Ventile zum Verhindern oder Reduzieren von Strömung in dem oder jedem Pfad.

Die Druckschrift EP 0 154 845 A2 offenbart weiterhin ein flaches Filterelement zur Filtration von Fluiden.

Es ist somit die Aufgabe der Erfindung, ein Prozessiersystem für biopharmazeutische und/oder chemische Prozesse bereitzustellen, das die Überwachung des Fluid in dem Prozessiersystems vereinfacht.

Diese Aufgabe wird durch ein modulares Prozessiersystem für biopharmazeutische und/oder chemische Prozesse gelöst, welches umfasst:
- zumindest eine Prozessiereinheit zur Durchführung eines Filtrationsschritts oder eine Chromatographieschritts in einem biopharmazeutischen oder chemischen Prozess;
- zumindest eine Adapterplatte, welche fluidisch mit der Prozessiereinheit unmittelbar oder mittelbar verbunden ist, wobei die Adapterplatte zumindest einen Adapterkanal aufweist, der von zumindest einem Fluidstrom durchströmbar ist, welcher zu der Prozessiereinheit strömt,
   wobei die Adapterplatte ferner zumindest ein als Mehrwegventil ausgebildetes Umlenkelement und/oder eine Pumpe und/oder zumindest ein Ventil aufweist;
   Abschlusshalterungen, zwischen welchen die zumindest eine Prozessiereinheit und die zumindest eine Adapterplatte sandwichartig gehalten werden; und
- ein externes Steuergerät;

wobei die Adapterplatte derart ausgebildet ist, dass der Fluidstrom zu der Prozessiereinheit mit Hilfe des zumindest einen als Mehrwegventil ausgebildeten Umlenkelements in dem Adapterkanal zumindest teilweise umlenkbar ist und/oder der Fluidstrom, bevorzugt dessen Druck, mit Hilfe des Ventils und/oder der Pumpe in dem Adapterkanal regelbar ist;
wobei in der Prozessiereinheit und/oder in der Adapterplatte jeweils zumindest ein Sensor eingebettet ist, um zumindest eine Eigenschaft des Fluidstroms in der Prozessiereinheit bzw. der Adapterplatte zu detektieren;
wobei das zumindest eine als Mehrwegventil ausgebildete Umlenkelement und/oder die Pumpe und/oder das zumindest eine Ventil mittels eines Aktuators steuerbar sind; und
wobei das externe Steuergerät derart mit dem zumindest einen Sensor (direkt oder indirekt) gekoppelt ist, dass Messdaten des zumindest einen Sensors ausgelesen werden können und basierend auf den ausgelesenen Messdaten das externe Steuergerät den Aktuator derart steuert, dass der Fluidstrom in der Prozessiereinheit und/oder der Adapterplatte von dem externen Steuergerät zentral geregelt werden kann.

Als "Prozessiereinheit" wird insbesondere eine Einheit verstanden, innerhalb der ein spezieller Prozessschritt für das gewünschte Verfahren ausgeführt wird. Insbesondere erfolgt innerhalb einer Prozessiereinheit eine Trennung von Komponenten eines Fluidstroms. Beispielsweise kann eine Prozessiereinheit eine Einheit zur Zellabtrennung (beispielsweise durch Tiefenfiltration), zur Sterilfiltration, für einen Chromatographieschritt, zur Virusinaktivierung oder zur Crossflow-Filtration sein.

Die Adapterplatte ist bezüglich des Fluidstroms vor der Prozessiereinheit geschaltet, so dass der Fluidstrom von der Adapterplatte zu der Prozessiereinheit strömt.

Durch die Adapterplatte, die einer Prozessiereinheit in einem modularen Prozessiersystem vorschaltbar ist, können erforderliche Anpassungen bezüglich des Fluidstroms vorgenommen werden. Dabei kann der Fluidstrom zumindest teilweise umgelenkt bzw. abgelenkt bzw. in der Flußrichtung geändert werden und/oder der Fluidstrom mit Hilfe der Adapterplatte, bevorzugt bzw. insbesondere dessen Druck, mit dem das Fluid auf die Prozessiereinheit trifft, geregelt oder gesteuert werden. Es können jedoch auch beide Anpassungen gleichzeitig innerhalb einer Adapterplatte vorgenommen werden. Ein Umlenken kann vorzugsweise mit Hilfe zumindest eines Umlenkelements erfolgen, das sich in oder an dem Adapterkanal befindet. Zusätzlich oder alternativ kann/können sich in dem Adapterkanal eine Pumpe und/oder zumindest ein Ventil befinden, über die/das der Druck des Fluidstroms in dem Adapterkanal geregelt oder gesteuert werden kann.

Somit kann eine Adapterplatte vor bzw. an einer Prozessiereinheit in einem Prozessiersystem angeordnet werden, um die nötigen Anpassungen bezüglich des Fluidstroms vornehmen zu können.

Die Vorschaltung einer Adapterplatte vor einer Prozessiereinheit in einem modularen Prozessiersystem bietet die Möglichkeit, einen Fluidstrom in einfacher Art und Weise der folgenden Prozessiereinheit so zuzuführen, wie dies für den folgenden Prozessschritt in dem Prozessiersystem benötigt wird. Aufgrund der Bauweise bietet die Adapterplatte in Kombination mit der Verschaltung einer Prozessiereinheit eine kompakte Bauweise, so dass der Bedarf an Stellfläche und Anlagenkomponenten reduziert werden kann. Entsprechend verringern sich die notwendigen Investitionen.

Weiterhin ist in dem Prozessiersystem in der Prozessiereinheit und/oder in der Adapterplatte zumindest ein Sensor eingebettet, der zumindest eine Eigenschaft des Fluidstroms detektieren kann. Hierdurch kann an bevorzugten Positionen Messungen an dem Fluidstrom vorgenommen werden und so der Fluidstrom permanent überwacht werden. Durch eine Einbettung des zumindest einen Sensors in die Prozessiereinheit und/oder Adapterplatte ist es insbesondere nicht notwendig, einen Sensor separat anzuschließen. Dieser ist werksseitig bereits in die Adapterplatte bzw. der Prozessiereinheit eingearbeitet bzw. in dieser vorgesehen und kann in vorteilhafter Weise vorinstalliert und/oder sterilisiert sein. Für den Benutzer entfallen damit insbesondere Arbeitsschritte einer Kalibrierung bzw. Sterilisation.

Die Messdaten des zumindest einen Sensors in der Prozessiereinheit und/oder der Adapterplatte sind von einem externen Steuergerät auslesbar. Weichen die Messdaten von vordefinierten optimalen Werten ab, kann das externe Steuergerät den Fluidstrom in dem Adapterkanal und/oder der Prozessiereinheit zentral regeln.

Das modulare Prozessiersystem benötigt somit nur ein externes Regel- bzw. Steuergerät, um Messdaten von dem Fluidstrom zu erhalten und den Fluidstrom in dem Prozessiersystem zu regeln.

Vorzugsweise umfasst das Prozessiersystem zumindest eine erste und zweite Prozessiereinheit, welche fluidisch miteinander verbunden sind,
wobei der zumindest eine Adapterkanal der Adapterplatte von zumindest einem Fluidstrom durchströmbar ist, welcher von der ersten Prozessiereinheit zu der zweiten Prozessiereinheit strömt; und
wobei die Adapterplatte derart ausgebildet ist, dass der Fluidstrom zwischen der ersten Prozessiereinheit und der zweiten Prozessiereinheit mit Hilfe des zumindest einen als Mehrwegventil ausgebildeten Umlenkelements in dem Adapterkanal zumindest teilweise umlenkbar ist und der Fluidstrom, bevorzugt dessen Druck, mit Hilfe des zumindest einen Ventils und/oder der Pumpe in dem Adapterkanal regelbar ist.

Die erste und zweite Prozessiereinheit können identisch ausgebildet sein oder zumindest teilweise unterschiedliche Eigenschaften aufweisen. Beispielsweise können die erste und zweite Prozessiereinheit in ihrer Größe variieren, können jedoch mit Hilfe der Adapterplatte dennoch in einfacher Weise miteinander verschaltet bzw. kombiniert werden. Vorzugsweise werden in einem Prozessiersystem unterschiedliche Prozessiereinheiten miteinander verschaltet, die beispielsweise unterschiedliche Trennmedien verwenden oder für unterschiedliche Prozessierstufen eines Verfahrens zuständig sind. Gleiche Prozessiereinheiten können dann zum Einsatz kommen, wenn eine Kapazitätserweiterung gewünscht wird.

Die Adapterplatte ist bezüglich des Fluidstroms zwischen der ersten Prozessiereinheit und der zweiten Prozessiereinheit geschaltet und verbindet somit die erste Prozessiereinheit und die zweite Prozessiereinheit fluidisch.

Durch die Adapterplatte, die (zumindest teilweise) zwischen zwei Prozessiereinheiten in einem modularen Prozessiersystem schaltbar ist, können erforderliche Anpassungen bezüglich des Fluidstroms vorgenommen werden, die es ermöglichen, Prozessiereinheiten (insbesondere Standard-Prozessiereinheiten) miteinander fluidisch zu verbinden. Dabei kann der Fluidstrom entsprechend zumindest teilweise umgelenkt bzw. abgelenkt bzw. in der Flußrichtung geändert werden und/oder der Fluidstrom mit Hilfe der Adapterplatte, bevorzugt bzw. insbesondere dessen Druck, mit dem das Fluid auf die zweite Prozessiereinheit trifft, geregelt oder gesteuert werden. Es können jedoch auch beide Anpassungen gleichzeitig innerhalb einer Adapterplatte vorgenommen werden.

Somit kann zumindest eine Adapterplatte zwischen zwei aufeinander folgenden Prozessiereinheiten in einem Prozessiersystem angeordnet werden, um die nötigen Anpassungen bezüglich des Fluidstroms vornehmen zu können.

Eine Verschaltung von Prozessschritten basierend auf einem modular aufgebauten Prozessiersystem mit mindestens einer Adapterplatte zwischen zwei Prozessiereinheiten ermöglicht in vorteilhafter Weise die Kombination von jeglichen Prozessschritten in einem Prozessiersystem, so dass der Bedarf an Stellfläche und Anlagenkomponenten reduziert werden kann. Entsprechend verringern sich die notwendigen Investitionen. Weiterhin wird insbesondere eine kompakte und/oder flexible Verschaltung für die kontinuierliche Betriebsweise in einem Prozessiersystem, inklusive der Überwachung mehrerer Grundoperationen, ermöglicht.

Weiterhin ist es bevorzugt, dass in der ersten und zweiten Prozessiereinheit und in der Adapterplatte jeweils zumindest ein Sensor eingebettet ist, um zumindest eine Eigenschaft des Fluidstroms in der ersten und zweiten Prozessiereinheit und der Adapterplatte zu detektieren.

Hierdurch wird eine kontinuierliche Überwachung des Fluidstroms über den Verlauf des Prozessiersystems gewährleistet. Mit Hilfe der Messdaten der Sensoren kann das Regel- bzw. Steuergerät an den verschiedenen Stellen in dem Prozessiersystem zumindest einen Parameter des Fluidstroms auswerten und unmittelbar Maßnahmen veranlassen, die entsprechenden Parameter des Fluidstroms zu korrigieren, sollte eine Abweichung von den vorgegebenen Grenzwerten vorliegen. Hierdurch kann in vorteilhafter Weise über den Verlauf des Prozessiersystems in das Prozessergebnis Einfluss genommen werden und Parameter des Fluidstroms, welche zu einem negativen Prozessergebnis führen würden in gewünschter Art und Weise beeinflusst werden. Wie bereits vorangehend beschrieben wurde, kann unter anderem die Pumpe und/oder das zumindest eine Ventil und/oder das zumindest ein Umlenkelement in der Adapterplatte entsprechend durch das Steuergerät gesteuert bzw. geregelt werden.

Vorzugsweise ist das externe Steuergerät derart mit den Sensoren gekoppelt, dass Messdaten der Sensoren ausgelesen werden können und basierend auf den ausgelesenen Messdaten der Fluidstrom in den Prozessiereinheiten und der Adapterplatte von dem externen Steuergerät zentral geregelt werden kann.

Es ist bevorzugt, dass die zumindest eine Prozessiereinheit und/oder die Adapterplatte jeweils zumindest einen Transponder aufweisen, der dazu ausgelegt ist, Messdaten eines entsprechenden Sensors an das externe Steuergerät zu übertragen; oder wobei die Sensoren der Prozessiereinheiten und/oder der Adapterplatte mit dem externen Steuergerät über ein Bussystem gekoppelt sind.

Als "Transponder" wird eine Übertragungseinheit verstanden, die die Messdaten des Sensors kabellos, optisch und/oder per Funk an das externe Steuergerät überträgt. Alternativ kann das Prozessiersystem ein Bussystem umfassen, über das der zumindest eine Sensor Messdaten an des externe Steuergerät übertragen kann.

Das zumindest eine Umlenkelement und/oder die Pumpe und/oder das zumindest eine Ventil ist/sind mittels eines Aktuators steuerbar.

Als "Aktuator" wird eine antriebstechnische Baueinheit verstanden, die Regelbefehle des externen Regel- bzw. Steuergeräts empfängt und die Befehle in eine mechanische Bewegung umsetzt. Diese kann ein Verstellen des zumindest einen Umlenkelements und/oder der Pumpe und/oder des Ventils umfassen. Die Befehle werden von dem externen Steuergerät ausgegeben.

Es werden somit nicht nur zentral Messdaten ausgelesen, sondern es kann auch zentral und automatisiert der Fluidstrom in dem Prozessiersystem geregelt werden.

Der Sensor kann dazu ausgebildet sein, einen Druck, einen Volumenstrom, einen UV-Wert, einen pH-Wert, eine Trübung und/oder eine Viskosität des Fluidstroms zu messen.

Somit können je nach Bedarf zumindest ein Parameter des Fluidstroms überwacht werden und gegebenenfalls Adaptierungen an dem Fluidstrom vorgenommen werden. Diese können durch das externe Steuergerät erfolgen und/oder das externe Steuergerät gibt eine Ausgabe (z.B. einen Alarm) an den Benutzer aus. Dieser kann gegebenenfalls weitere Adaptierungen vornehmen, sollte beispielsweise der pH-Wert des Fluids zu hoch sein.

Vorzugsweise weisen der zumindest eine Sensor, das zumindest eine als Mehrwegventil ausgebildete Umlenkelement, die Pumpe und/oder das zumindest eine Ventil jeweils einen Akku auf.

Mit Hilfe eines Akkus erhält der Sensor die erforderliche Energie, um Messungen vornehmen zu können, das Umlenkelement bzw. das Ventil kann durch die Energie im Bedarfsfall neu eingestellt werden und die Pumpe kann durch die Energie des Akkus im Bedarfsfall die erforderliche Pumpleistung abgeben.

Insbesondere bietet der Akku den Vorteil, dass keine Kabel nach außen geführt werden und von dem Benutzer angeschlossen werden muss. Die jeweilige Komponente ist durch einen Akku bereits mit ausreichend Energie versorgt. Insbesondere kann der Akku auch induktiv geladen werden.

Alternativ oder ergänzend kann der zumindest eine Sensor, das zumindest eine Umlenkelement, das zumindest eine Ventil und/oder die Pumpe eine kabelgebundene Stromversorgung aufweisen.

Eine kabelgebundene Energieversorgung bietet in vorteilhafter Weise insbesondere für den Fall einer längeren Betriebsdauer des Prozessiersystems die erforderliche Energie für die genannten Komponenten.

Vorzugsweise weist das Prozessiersystem eine zentrale Stromversorgung für den zumindest einen Sensor, das zumindest eine als Mehrwegventil ausgebildete Umlenkelement, das zumindest eine Ventil und/oder die Pumpe auf,
wobei die zumindest Prozessiereinheit und die zumindest eine Adapterplatte Teilabschnitte der Stromversorgung aufweisen, die wenn zusammengefügt, die zentrale Stromversorgung bilden.

Mit anderen Worten ist in der Prozessiereinheit und in der Adapterplatte jeweils ein Teilabschnitt der kabelgebundene Stromversorgung (Kabel) integriert. Wird das Prozessiersystem zusammengesetzt, werden die Kabelabschnitte der zumindest einen Prozessiereinheit und der zumindest einen Adapterplatte miteinander verbunden. Hierdurch muss nur an einer Stelle dem Prozessiersystem Strom zugeführt werden. Durch die zentrale Stromversorgung, die durch das Prozessiersystem verläuft, können alle Komponenten in dem Prozessiersystem, wie z.B. die Sensoren, mit Energie versorgt werden.

Es ist bevorzugt, dass der zumindest eine Sensor, das zumindest eine als Mehrwegventil ausgebildete Umlenkelement, das zumindest eine Ventil und die Pumpe als Einweg-Elemente ausgebildet sind.

Als "Einweg" wird hierbei verstanden, dass der Sensor, das Umlenkelement und die Pumpe zusammen mit der Prozessiereinheit bzw. der Adapterplatte, je nachdem in welches Element sie eingebettet sind, nach deren Verwendung entsorgt werden können. Eine Reinigung und Wiederaufbereitung für einen weiteren Einsatz in einer neuen Prozessiereinheit oder Adapterplatte wird hierdurch vermieden.

Weiterhin wird die zugrunde liegende Aufgabe durch ein Verfahren zum zentralen Regeln eines modularen Prozessiersystems für biopharmazeutische und/oder chemische Prozesse gelöst. Das Verfahren umfasst die Schritte:
- Bereitstellen zumindest einer Prozessiereinheit zur Durchführung eines Filtrationsschritts oder eines Chromatographieschritts in einem biopharmazeutischen oder chemischen Prozess;
- Bereitstellen zumindest einer Adapterplatte, welche zumindest einen Adapterkanal aufweist, der von zumindest einem Fluidstrom durchströmbar ist, wobei die Adapterplatte ferner zumindest ein als Mehrwegventil ausgebildetes Umlenkelement und/oder zumindest ein Ventil und/oder eine Pumpe aufweist, wobei das zumindest eine als Mehrwegventil ausgebildete Umlenkelement und/oder die Pumpe und/oder das zumindest eine Ventil mittels eines Aktuators steuerbar ist/sind;
- Bereitstellen eines externen Steuergeräts;
- Mittelbares oder unmittelbares Verbinden der Adapterplatte mit der Prozessiereinheit, so dass der Fluidstrom von der Adapterplatte zu der Prozessiereinheit strömen kann;
- Bereitstellen von Abschlusshalterungen und Anordnen der zumindest einen Prozessiereinheit und der zumindest einen Adapterplatte zwischen den Abschlusshalterungen, so dass die zumindest eine Prozessiereinheit und die zumindest eine Adapterplatte sandwichartig zwischen den Abschlusshalterungen gehalten werden;
- Detektieren zumindest einer Eigenschaft des Fluidstroms in der Prozessiereinheit und/oder der Adapterplatte mittels zumindest eines Sensors, der in der Prozessiereinheit und/oder der Adapterplatte eingebettet ist; und
- Koppeln des externen Steuergeräts mit dem zumindest einen Sensor, so dass Messdaten des zumindest einen Sensors ausgelesen werden können; und
- Koppeln des externen Steuergeräts mit dem zumindest einen als Mehrwegventil ausgebildeten Umlenkelement und/oder der Pumpe und/oder dem zumindest einen Ventil in der Adapterplatte, so dass basierend auf den ausgelesenen Messdaten das externe Steuergerät den Aktuator derart steuert, dass der Fluidstrom in der Prozessiereinheit und/oder der Adapterplatte von dem externen Steuergerät zentral geregelt werden kann;

wobei mit Hilfe des zumindest einen als Mehrwegventil ausgebildeten Umlenkelements in dem Adapterkanal der Fluidstrom zumindest teilweise umlenkbar ist, und/oder
wobei der Fluidstrom, bevorzugt dessen Druck, mit Hilfe des zumindest einen Ventils und/oder der Pumpe in dem Adapterkanal regelbar ist.

Vorzugsweise umfasst das Prozessiersystem zumindest eine erste und zweite Prozessiereinheit; und
wobei die erste und zweite Prozessiereinheit mittels der Adapterplatte derart miteinander gekoppelt werden, dass der Fluidstrom von der ersten Prozessiereinheit zu der zweiten Prozessiereinheit strömen kann.

Es ist bevorzugt, dass in der ersten und zweiten Prozessiereinheit und in der Adapterplatte jeweils zumindest ein Sensor eingebettet ist;
wobei die Sensoren zumindest eine Eigenschaft des Fluidstroms in der ersten und zweiten Prozessiereinheit und der Adapterplatte detektieren; und
wobei die Sensoren mit dem externen Steuergerät gekoppelt werden, so dass Messdaten der Sensoren ausgelesen werden können und basierend auf den ausgelesenen Messdaten der Fluidstrom in den Prozessiereinheiten und der Adapterplatte von dem externen Steuergerät zentral geregelt werden kann.

Diese und andere Ziele, Merkmale und Vorteile der vorliegenden Erfindung werden durch das Studium der folgenden detaillierten Beschreibung bevorzugter Ausführungsformen und der beigefügten Zeichnungen klarer. Weiterhin wird darauf hingewiesen, dass, obwohl Ausführungsformen separat beschrieben werden, einzelne Merkmale dieser Ausführungsformen zu zusätzlichen Ausführungsformen kombiniert werden können.
- Fig. 1a)-f): zeigen einen grundlegenden Aufbau verschiedener Prozessiereinheiten;
- Fig. 2a): zeigt ein Prozessiersystem mit zwei Prozessiereinheitengruppen gemäß einer Ausführungsform, die mit Hilfe einer Adapterplatte parallel verschaltet sind;
- Fig. 2b): zeigt das Prozessiersystem aus Fig. 2a), in dem zwei Prozessiereinheitengruppen mit Hilfe der Adapterplatte seriell verschaltet sind;
- Fig. 2c): zeigt das Prozessiersystem aus Fig. 2b, in dem die einzelnen Prozessiereinheiten mittels Abschlusshalterungen zusammengehalten sind;
- Fig. 3a): zeigt eine Schnittansicht durch eine Adapterplatte aus den Figuren 2a) und 2b) mit einem Mehrwegventil;
- Fig. 3b): zeigt das Mehrwegventil aus Figur 3a);
- Fig. 4a): zeigt eine Schnittansicht einer zweiteiligen Adapterplatte mit einem Sensor gemäß einer weiteren Ausführungsform;
- Fig. 4b): zeigt eine perspektivische Ansicht der Adapterplatte aus Fig. 4a);
- Fig. 5a): zeigt eine Schnittansicht einer Adapterplatte gemäß einer weiteren Ausführungsform mit einer Hilfsabzweigung, in der ein Adapterplatten-Sensor integriert ist;
- Fig. 5b): zeigt eine perspektivische Ansicht der Adapterplatte aus Fig. 5a);
- Fig. 6a): zeigt ein Prozessiersystem mit einer Adapterplatte gemäß einer weiteren Ausführungsform, die zwei Hilfsaus- bzw. -zugänge aufweist;
- Fig. 6b): zeigt eine Ausführungsform einer Adapterplatte aus Fig. 6a), in der ein Hilfszugang zur Fluidverdünnung verwendet wird;
- Fig. 6c): zeigt eine Ausführungsform einer Adapterplatte aus Fig. 6a), in der Hilfszugänge zur Virusinaktivierung verwendet werden;
- Fig. 7: zeigt ein Prozessiersystem mit einer Adapterplatte gemäß einer weiteren Ausführungsform, in der eine Pumpe integriert ist;
- Fig. 8a): zeigt eine Schnittansicht durch eine Adapterplatte mit einer Kolbenpumpe in einer Ansaugposition;
- Fig. 8b): zeigt eine Schnittansicht der Adapterplatte aus Figur 8a) in einer Hubposition;
- Fig.9a): zeigt eine Explosionsansicht einer Adapterplatte mit einer Peristaltikpumpe gemäß einer weiteren Ausführungsform;
- Fig. 9b): zeigt eine Schnittansicht der Adapterplatte aus Fig. 9a);
- Fig. 10a): zeigt eine Prozessiereinheit gemäß einer Ausführungsform, in der ein Sensor eingebettet ist, dessen Daten kabellos an eine externe Steuereinheit übertragen werden; und
- Fig. 10b): zeigt ein Prozessiersystem gemäß einer Ausführungsform mit Sensoren, die über ein Bussystem an das externe Steuergerät übertragen werden.

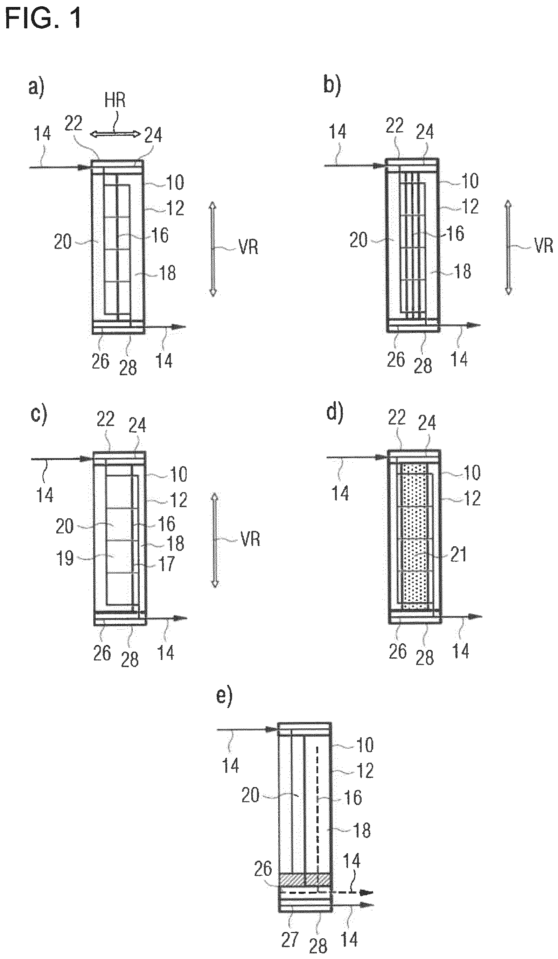

Für biopharmazeutische und chemische Prozesse bestehen verschiedene Prozessiereinheiten, die im Rahmen der vorliegenden Erfindung zum Einsatz kommen können. Die **Figuren 1a) bis 1e****)** zeigen einen grundlegenden Aufbau verschiedener Prozessiereinheiten, die im Rahmen der vorliegenden Erfindung verwendet werden können. Es handelt sich hierbei um eine Auswahl, jedoch keine abschließende Liste.

**Fig. 1a****)** zeigt einer Prozessiereinheit 10, die dazu verwendet werden kann, einen bestimmten Filtrationsschritt in einem biopharmazeutischen oder chemischen Prozess zu übernehmen. Hierzu weist die Prozessiereinheit 10 ein Prozessiergehäuse 12 auf, das von einem Fluidstrom 14 durchströmbar ist. Der Fluidstrom 14 umfasst das zu filtrierende Medium. In dem Prozessiergehäuse 12 ist zumindest ein Filtermedium 16 angeordnet, das ein poröses Material umfasst, das danach gewählt bzw. verwendet wird, welche Partikel bzw. welche Stoffe mit Hilfe der Prozessiereinheit 10 aus dem Fluidstrom 14 herausgefiltert werden soll. Beispielsweise kann es sich bei dem Filtermedium 16 um einen Virusfilter, einen Sterilfilter, einen Tiefenfilter oder einen Membranadsorber handeln. Das Filtermedium 16 ist vorzugsweise als Filtermatte bzw. -membran bzw. - membranschicht(en) ausgebildet. In einer bevorzugten Ausführungsform kann das Filtermedium 16 aus mehreren Schichten bestehen. Üblicherweise ist das Filtermedium 16 im Wesentlichen in vertikaler Richtung VR in dem Prozessiergehäuse 12 angeordnet. In dem Prozessiergehäuse 12 trennt das Filtermedium 16 eine Filtratseite 18 von einer Retentatseite 20. Das Filtermedium 16 ist fluidisch durchlässig, wobei filtermediumsspezifische Stoffe das Filtermedium 16 nicht passieren können. Da der Fluidstrom 14 bestimmungsgemäß von der Retentatseite 20 zur Filtratseite 18 gerichtet ist, verbleiben diese filtermediumsspezifische Stoffe auf der Retentatseite 20 und/oder im Filtermedium 16, gelangen jedoch im Wesentlichen nicht auf die Filtratseite 18 der Prozessiereinheit 10. Zwischen der Retentatseite 20 und der Filtratseite 18 herrscht eine Fluiddruckdifferenz in Abhängigkeit von dem angelegten Fluiddruck und/oder der Durchlässigkeit des Filtermediums 16.

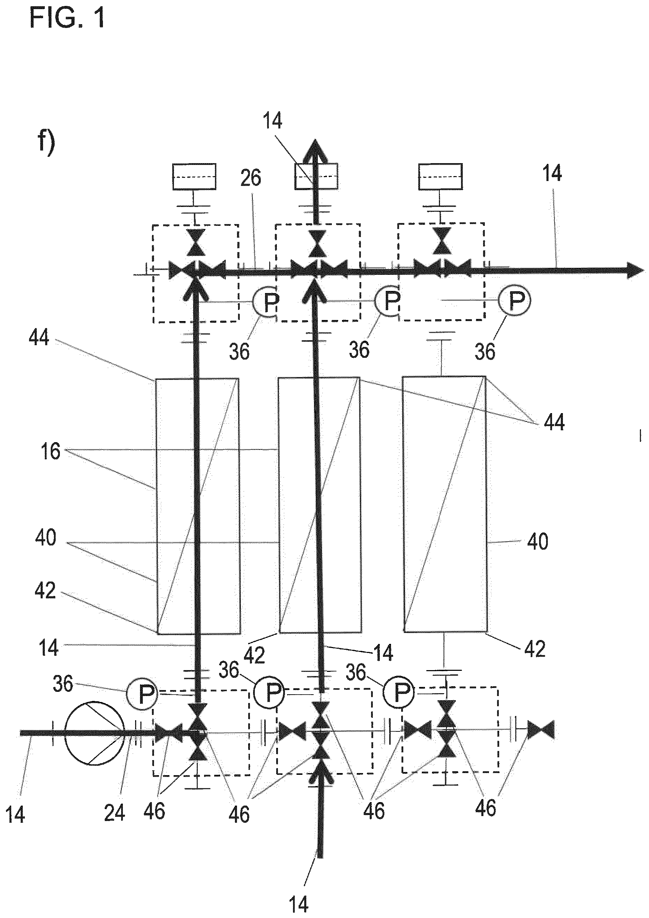

An einem vorzugsweise oberen Ende 22 des Prozessiergehäuses 12 befindet sich zumindest ein Zulaufkanal 24. Dieser erstreckt sich vorzugsweise in im Wesentlichen horizontaler Richtung HR und speist die Prozessiereinheit 10 mit dem zu filtrierenden Medium. Wie in Figur 1 mit Hilfe eines Pfeils gekennzeichnet, strömt der Fluidstrom 14 über den Zulaufkanal 24 in das Prozessiergehäuse 12. In Figur 1 bedeutet das, dass ein Fluidstrom 14 von links in das Prozessiergehäuse 12 strömt. Zumindest ein Teil des Fluidstroms 14 strömt anschließend von der Retentatseite 20 durch das Filtermedium 16 zur Filtratseite 18. Ist die Prozessiereinheit 10 mit einer weiteren Prozessiereinheit (hier nicht gezeigt) parallel verschaltet, strömt ein weiterer Teil des Fluidstroms 14 direkt zu der weiteren Prozessiereinheit ohne das Filtermedium 16 zu durchdringen. Das heißt, dieser Teil des Fluidstroms 14 strömt in den Zulaufkanal 24 der weiteren (nicht gezeigten) Prozessiereinheit. Der Fluidstrom 14, der das Filtermedium 16 durchdrungen hat ("Filtrat"), strömt anschließend in einen Ablaufkanal 26 am vorzugsweise unteren Ende 28 des Prozessiergehäuses 12 und strömt von dort aus dem Prozessiergehäuse 12. Der Ablaufkanal 26 erstreckt sich vorzugsweise ebenfalls im Wesentlichen horizontaler Richtung HR in dem Prozessiergehäuse 12. Das Filtrat, das das Prozessiergehäuse 12 verlässt, kann anschließend in den Ablaufkanal 26 einer weiteren Prozessiereinheit (hier nicht gezeigt) strömen (Parallelschaltung) und/oder zur weiteren Prozessierung in den Zulaufkanal 24 der weiteren Prozessiereinheit (Serienschaltung) strömen.

**Fig. 1b****)** zeigt die Prozessiereinheit 10 aus Fig. 1a), die sich lediglich von dieser unterscheidet, indem das Filtermedium 16 mehrlagig ausgebildet ist.

**Fig. 1c****)** zeigt eine Prozessiereinheit 10, die grundsätzlich ähnlich zu der Prozessiereinheit 10 aus Fig. 1a) ausgebildet ist, sich jedoch in der Art der Filtration unterscheidet. Es werden im Folgenden daher nur die Teile der Prozessiereinheit 10 aus Fig. 1c) beschrieben, die sich von der Prozessiereinheit 10 aus Fig. 1a) unterscheiden.

Im Speziellen ist die Prozessiereinheit aus Fig. 1c) zur Anschwemmfiltration aus gebildet. Dazu ist das Filtermedium 16 als Anschwemmfilter ausgebildet. Das Filtermedium 16 umfasst hier einen Filterträger 17, der vorzugsweise in vertikaler Richtung VR in dem Prozessiergehäuse 12 angeordnet ist und der relativ grob ausgebildet ist. Das Anschwemmmittel wird üblicherweise vor Einbringen in den Filter mit dem Fluid gemischt. Damit wird der Aufbau eines Filterkuchens (hier nicht gezeigt) ermöglicht. Der Filterträger 17 wird so gewählt, dass zumindest ein Filterhilfsmittel zurückgehalten wird. Um in dem Prozessiergehäuse 12 entsprechend Platz für den Filterkuchen bereitzustellen, ist auf der Retentatseite 20 ein Leerraum 19 ausgebildet.

**Fig. 1d****)** zeigt eine weitere Prozessiereinheit 10. Diese ist ähnlich zu der Prozessiereinheit 10 aus Fig. 1a) aufgebaut, weist jedoch anstatt eines Filtermediums 16 ein Schüttgut 21 auf. Im Speziellen kann es sich bei dem Schüttgut 21 um Gele oder Aktivkohle handeln, so dass sich die Prozessiereinheit 10 aus Fig. 1c) zur Chromatographie eignet.

Im Rahmen der Chromatographie können Stoffgemische aufgetrennt werden. Das Schüttgut 21 dient dabei als stationäre Phase, welche unbeweglich in der Prozessiereinheit 10 angeordnet ist. Ein Substanzgemisch wird mit Hilfe einer mobilen Phase (z.B. Wasser) zu der stationären Phase transportiert. Durch eine Wechselwirkung der stationären Phase mit einzelnen Substanzen in der mobilen Phase kann die Durchflusszeit der entsprechenden Substanz durch die Prozessiereinheit 10 verzögert werden, so dass eine Auftrennung von Substanzen ermöglicht wird.

**Fig. 1e** zeigt eine weitere Prozessiereinheit 10. Diese ist ebenfalls ähnlich zu der Prozessiereinheit 10 aus Fig. 1a) aufgebaut, unterscheidet sich jedoch darin, dass ein zweiter Ablaufkanal 27 bereitgestellt ist. Hierdurch eignet sich diese Prozessiereinheit 10 zur Tangentialflussfiltration bzw. Crossflowfiltration. Hierbei wird eine zu filtrierende Suspension mit einer hohen Geschwindigkeit parallel zu dem Filtermedium 16 gepumpt und das Filtrat quer zur Fließrichtung abgezogen. Das Filtrat kann dann über einen der Ablaufkanäle 26 abgeführt werden. Der nicht das Filtermedium 16 durchdringende Anteil des Fluidstroms 14, also das Retentat, kann über den zweiten Ablaufkanal 27 aus der Prozessiereinheit 10 abgeführt werden. Je nach Wunsch kann das Filtrat oder das Rententat aus der Crossflowfiltration im Rahmen des später beschriebenen Prozessiersystems weiter prozessiert werden.

Während das Filtermedium 16 in den Figuren 1a) bis 1e) als Flachfilter ausgebildet sind, kann das Filtermedium 16 alternativ eine Filterkerze bzw. Filterkapsule 40 sein, wie dies in **Figur 1f****)** gezeigt ist.

Während Flachfilter flach ausgebildet sind und sich in einer Ebene erstrecken, ist eine Filterkerze 40 zylindrisch geformt. Vorzugsweise umfasst das Filtermedium 16 ein plissiertes Filtermaterial, welches mehrlagig zu der zylinderischen Filterkerze 40 geformt ist. Um die notwendige Stabilität der Filterkerze 40 zu gewährleisten, umfasst die Filterkerze 40 vorzugsweise einen zylindrischen Stützkern, welcher das Filtermedium 16 von innen stützt und einen starren Außenkäfig, welcher das Filtermedium 16 von außen umschließt und dieses von außen stützt. Sowohl der Stützkern als auch der Außenkäfig sind jedoch derart ausgebildet, dass ein Fluidstrom durch diese hindurch zugelassen wird.

Figur 1f) zeigt schematisch allgemein einen Fluidstrom 14 durch die Filterkerze 40 hindurch. Der Fluidstrom 14 kann dabei derart zugeführt werden, dass der Fluidstrom 14 von einer Innenseite der Filterkerze 40 zu einer Außenseite der Filterkerze 40 strömt oder von der Außenseite der Filterkerze 40 zu der Innenseite der Filterkerze 40. Die Zufuhrrichtung wird dabei durch eine Endkappe beeinflusst, welche den zylindrischen Körper der Filterkerze 40 an eine Endseite verschließt.

Soll die Filterkerze 40 von innen nach außen durchströmt werden, wird der Fluidstrom 14 über eine erste Seite 42 der Filterkerze 40 einem Innenraum der Filterkerze 40 zugeführt. Die zweite, entgegengesetzte Seite 44 der Filterkerze 40 ist hier jedoch durch die Endkappe verschlossen, so dass das zugeführte Fluid dazu gezwungen wird, das Filtermedium 16 der Filterkerze 40 von innen nach außen zu durchströmen.

Soll die Filterkerze 40 alternativ von außen nach innen durchströmt werden, ist die Filterkerze 40 an dem ersten Ende 42 durch eine Endkappe verschlossen, so dass das Fluid von außen nach innen strömt. Das zweite Ende 44 der Filterkerze 40 ist hingegen hier offen.

Die Filterkerze 40 selbst ist in einem flachen Prozessiergehäuse 12 eingesetzt bzw. verbaut. Vorzugsweise weist das Prozessiergehäuse 12 in seinem Inneren Wandungen auf, welche den Fluidstrom 14 entsprechend leiten, so dass der Fluidstrom 14 der Filterkerze 40 in gewünschter Weise zugeführt wird. Soll der Fluidstrom 14 beispielsweise von innen nach außen an der Filterkerze 40 geführt werden, können Wandungen vorgesehen sein, die einen Fluidstrom 14 in das Innere der Filterkerze 40 leiten. Ein Kontakt des zu filtrierenden Mediums mit einer Außenseite der Filterkerze 40 kann hierdurch vermieden werden. Gleichzeitig wird vermieden, dass das filtrierte Medium mit dem zu filtrierenden Medium in Kontakt tritt.

Soll der Fluidstrom 14 von außen nach innen an der Filterkerze 40 strömen, ist vorzugsweise ebenfalls eine entsprechende Wandung benachbart zu der zweiten Seite 44 der Filterkerze 40 vorgesehen. Diese verhindert, dass das filtrierte Medium, welches an der zweiten Seite 44 der Filterkerze 40 austritt, nicht mit dem zu filtrierenden Medium an der Außenseite der Filterkerze 40 in Kontakt tritt.

Die Filterkerze 40 kann vertikal oder horizontal in dem Prozessiergehäuse 12 eingebaut werden. Der Zulaufkanal 24, über den das zu filtrierende Medium in das Prozessiergehäuse 12 strömt, ist hier entsprechend ausgebildet, so dass die Filterkerze bezüglich ihrer vertikalen oder horizontalen Ausrichtung entsprechend durchströmt werden kann. Gleiches gilt für den Ablaufkanal 26, über den das filtrierte Fluid aus dem Prozessiergehäuse 12 ausströmen kann.

Wie in Figur 1f) gezeigt ist, können in einem Prozessiergehäuse 12 mehrere Filterkerzen 40 parallel angeordnet werden. Die Zuführung und Abführung des Fluidstroms 14 von und zu der jeweiligen Filterkerze 40 erfolgt wie bereits oben bezüglich einer Filterkerze 40 beschrieben wurde. Ergänzend kann jedoch zwischen den einzelnen Filterkerzen 40 eine Trennwand vorgesehen sein, so dass die Filterkerzen 40 voneinander separiert sind.

In dem Zulaufkanal 24 und/oder in dem Ablaufkanal 26 kann zumindest ein Filterkerzenventil 46 vorgesehen sein. In dem Zulaufkanal 24 kann das Filterkerzenventil 46 dazu ausgelegt sein, ein Weiterströmen in den Zulaufkanal 24 zu ermöglichen oder zu verhindern. Weiterhin kann ein Filterkerzenventil 46 dazu ausgelegt sein, ein Einströmen bzw. Zuströmen des Fluidstroms 14 zu einer bzw. in eine Filterkerze 40 zu ermöglichen oder zu blockieren. Darüber hinaus, kann, wie in Figur 1f) gezeigt, ein Filterkerzenventil 46 dazu auslegt sein, einen Zugang von außen in den Zulaufkanal 24 zu gewähren, so dass Additive oder ein weitere Fluidstrom 14 in den Zulaufkanal 24 einströmen kann.

In dem Ablaufkanal 26 kann das Filterkerzenventil 46 dazu ausgelegt sein, ein Weiterströmen in den Ablaufkanal 26 zu ermöglichen oder zu verhindern. Weiterhin, kann, wie in Figur 1f) gezeigt, ein Filterkerzenventil 46 dazu auslegt sein, einen zusätzlichen Ausgang aus dem Ablaufkanal 26 zu gewähren, so dass zumindest ein Teil des Fluidstrom 14 aus dem Ablaufkanal 26 ausströmen kann.

Die Filterkerzenventile 46 sind insbesondere für Integritätstests vorteilhaft. Hier können mit Hilfe der Filterkerzenventile 46 Filterkerzen 40 separat einem Integritätstest unterzogen werden.

In das Prozessiergehäuse 12 kann zumindest ein Prozessiereinheiten-Sensor 36 eingebettet sein. Dieser ist derart positioniert, dass ein gewünschter Parameter des Fluidstroms 14 in der Prozessiereinheit 10 überwacht bzw. gemessen werden kann. An späterer Stelle wird der Prozessiereinheiten-Sensor 36 hierzu noch detaillierter beschrieben.

Für den Integritätstest kann der Prozessiereinheiten-Sensor 36 insbesondere dazu ausgelegt sein, den Druck des Fluidstroms 14 zu detektieren, wie dies in Figur 1f) gezeigt ist. Vorteilhafterweise befindet sich vor und nach einer Filterkerze 40 ein Prozessiereinheiten-Sensor 36, so dass ein Druckunterschied des Fluidstroms 14 detektiert werden kann.

Insbesondere für Filtrationsanwendungen, in denen hohe Drücke auf das Filtermedium 16 einwirken, eignen sie Filterkerzen 40 besonders.

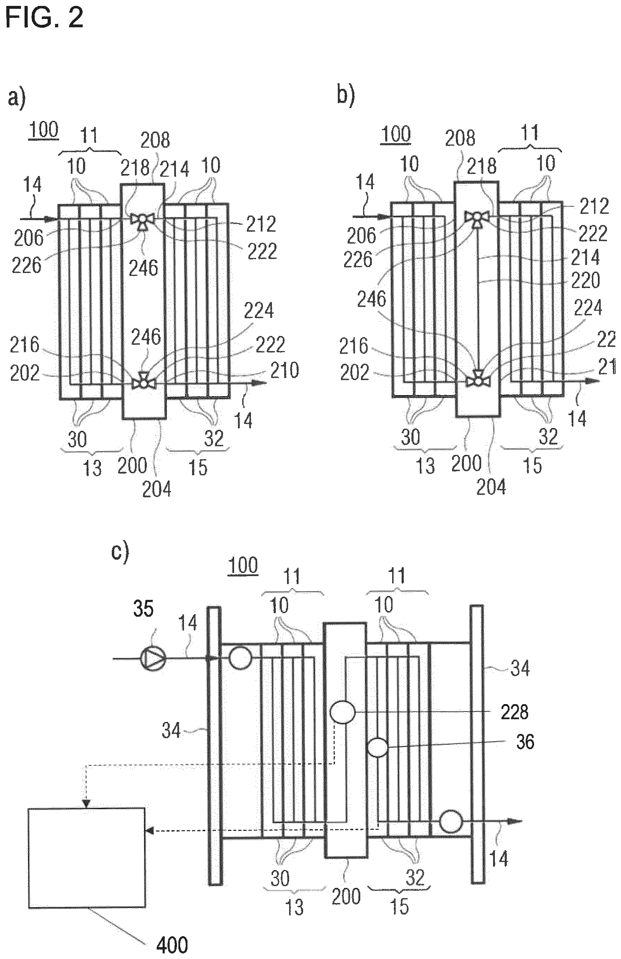

**Figuren 2a)** und **2b****)** zeigen jeweils Prozessiersysteme 100 mit einer Vielzahl von den in den Figuren 1a) bis 1e) beschriebenen Prozessiereinheiten 10. Prozessiereinheiten 10, die direkt miteinander gekoppelt sind, bilden eine Prozessiereinheitengruppe 11. Wie in den Figuren 2a) und 2b) gezeigt, umfassen die Prozessiersysteme 100 jeweils eine erste Prozessiereinheitengruppe 13 und eine zweite Prozessiereinheitengruppe 15, die mit Hilfe einer Adapterplatte 200 gemäß einer Ausführungsform der Erfindung gekoppelt sind. Die in den Figuren 2a) und 2b) direkt gekoppelten Prozessiereinheiten 10 sind als parallel verschaltete Einheiten gezeigt, können jedoch auch seriell verschaltet sein. Die beiden Prozessiereinheitengruppen 11, die mit Hilfe der Adapterplatte 200 gekoppelt sind, können durch die Adapterplatte 200 sowohl parallel (siehe Figur 2a)) als auch seriell (siehe Figur 2b)) verschaltet sein. Optional weist die Adapterplatte 200 zumindest ein später näher beschriebenes Umlenkelement auf, mit dem zwischen diesen Verschaltungsweisen gewechselt werden. Dies erfolgt in einfacher Weise und ohne konstruktive Änderungen in dem Prozessiersystem 100. Durch einfaches Umschalten der ein oder mehreren Umlenkelemente, kann zwischen einer parallelen und seriellen Verschaltung der Prozessiereinheitengruppen 11 gewechselt werden. Figur 2a) zeigt eine parallele Verschaltung von Prozessiereinheitengruppen 11, die durch die Adapterplatte 200 gekoppelt sind. Figur 2b) zeigt eine serielle Verschaltung der beiden Prozessiereinheitengruppen 11, die durch die Adapterplatten 200 gekoppelt sind. Obwohl die Figuren 2a) und 2b) Prozessiereinheitengruppen 11 zeigen, die durch die Adapterplatte 200 gekoppelt sind, kann die Adapterplatte 200 auch dazu genutzt werden, nur eine Prozessiereinheit 10 mit einer Prozessiereinheitengruppe 11 oder zwei einzelne Prozessiereinheiten 10 miteinander zu koppeln.

Die Adapterplatte 200 ist vorzugsweise im Wesentlichen plattenförmig ausgebildet und ist von einem Fluidstrom 14 durchströmbar. Vorzugsweise ist die Adapterplatte 200 als Einweg-Bauteil ausgeführt, wobei die Materialeigenschaften vorzugsweise derart gewählt sind, dass eine Sterilisationsmethode wie beispielsweise Gammabestrahlung, Autoklavieren, eine Durchströmung mit Gas wie Ethylenoxid und/oder Heißdampf angewandt werden kann. Insbesondere kann die Adapterplatte aus Kunststoff hergestellt sein. Prozessiereinheiten 10, die in Strömungsrichtung vor einer Adapterplatte 200 angeordnet sind werden als erste Prozessiereinheiten 30 bezeichnet, während Prozessiereinheiten 10, die in Strömungsrichtung nach einer Adapterplatte 200 angeordnet sind als zweite Prozessiereinheiten 32 bezeichnet werden. Mit anderen Worten besteht die erste Prozessiereinheitengruppe 13 aus ersten Prozessiereinheiten 30 und die zweite Prozessiereinheitengruppe 15 aus zweiten Prozessiereinheiten 32, wie in den Figuren 2a) und 2b) gezeigt. Die Adapterplatte 200 ist zwischen der ersten Prozessiereinheitengruppe 13 und der zweiten Prozessiereinheitengruppe 15 angeordnet und verbindet diese fluidisch. Die Adapterplatte 200 umfasst zumindest eine Zutrittsöffnung, über die der Fluidstrom 14 in die Adapterplatte 200 strömen kann. Wie in Figur 2a) gezeigt, weist die Adapterplatte 200 zwei Zutrittsöffnungen auf, nämlich eine erste Zutrittsöffnung 202 am vorzugsweise unteren Ende 204 der Adapterplatte 200, welche mit dem Ablaufkanal 26 der ersten Prozessiereinheit 30 gekoppelt ist, die direkt vor der Adapterplatte 200 angeordnet ist, und eine zweite Zutrittsöffnung 206 am vorzugsweise oberen Ende 208 der Adapterplatte 200, welche mit dem Zulaufkanal 24 dieser ersten Prozessiereinheit 30 gekoppelt ist. Mit anderen Worten kann ein Fluidstrom 14 von der genannten ersten Prozessiereinheit 30 über die erste und zweite Zutrittsöffnung 202 und 206 in die Adapterplatte 200 strömen bzw. fließen. Weiterhin weist die Adapterplatte 200 zumindest eine Austrittsöffnung auf, über die der Fluidstrom 14 aus der Adapterplatte 200 austreten kann. Wie in Figur 2a) gezeigt, weist die Adapterplatte 200 zwei Austrittsöffnungen auf, nämlich eine erste Austrittsöffnung 210, welche vorzugsweise am bzw. nahe einem unteren Ende 204 der Adapterplatte 200 angeordnet ist und mit dem Ablaufkanal 26 der zweiten Prozessiereinheit 32, die direkt nach der Adapterplatte 200 angeordnet ist, gekoppelt ist bzw. werden kann, und eine zweite Austrittsöffnung 212, welche vorzugsweise am bzw. nahe einem oberen Ende 208 der Adapterplatte 200 angeordnet ist und mit dem Zulaufkanal 24 dieser zweiten Prozessiereinheit 32 gekoppelt ist bzw. werden kann. Innerhalb der Adapterplatte 200 erstreckt sich zumindest ein Adapterkanal 214, der die Zutritts- und Austrittsöffnungen miteinander fluidisch verbindet. Im Speziellen weist der Adapterkanal 214 einen ersten Kanalbereich 216 auf, welcher sich zwischen der ersten Zutrittsöffnung 202 und der ersten Austrittsöffnung 210 erstreckt. Ein zweiter Kanalbereich 218 erstreckt sich zwischen der zweiten Zutrittsöffnung 206 und der zweiten Austrittsöffnung 212. Der erste und zweite Kanalbereich 216 und 218 wird über einen Verbindungskanalbereich 220 fluidisch verbunden.

Der oben beschriebene Aufbau des Adapterkanals 214 ist insbesondere dann vorteilhaft, wenn durch das zumindest eine Umlenkelement ein Umschalten zwischen einer seriellen und parallelen Verschaltung zweier Prozessiereinheiten 10 gewechselt werden kann. Weist die Adapterplatte 200 kein Umlenkelement auf, kann auch lediglich ein vorgegebener Fluidpfad in der Adapterplatte 200 vorgesehen sein. Alternativ kann der Adapterkanal 214 wie oben beschrieben aufgebaut sein, jedoch sind Abschnitte des Adapterkanals 214 fest verschlossen, so dass ein vorgegebener Fluidpfad durch die Adapterplatte 200 entsteht.

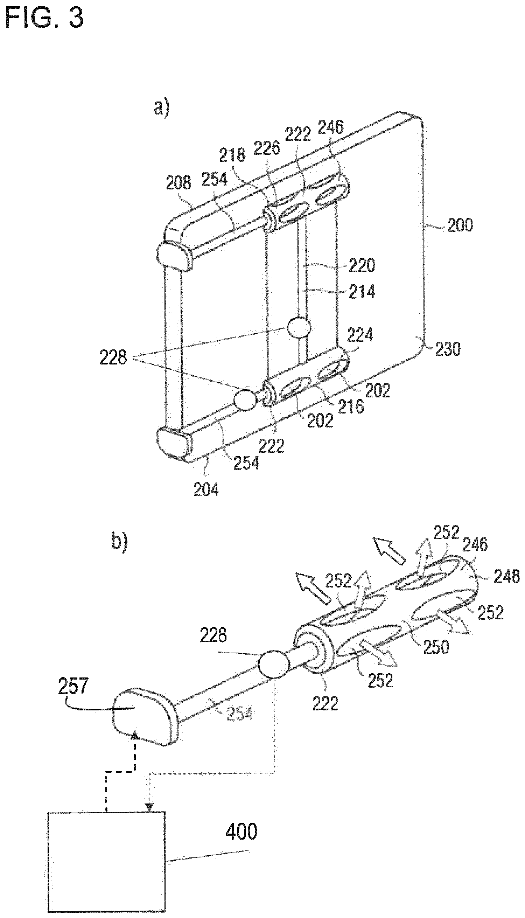

Wie in den Figuren 2a) und 2b) gezeigt, sind in dem Adapterkanal 214 zumindest zwei Umlenkelemente angeordnet. Im Speziellen handelt es sich hierbei um Umlenkventile 222. In einer bevorzugten Ausführungsform ist ein erstes Umlenkventil 224 in dem ersten Kanalbereich 216 und ein zweites Umlenkventil 226 in dem zweiten Kanalbereich 218 angeordnet. Konkret sind die Umlenkventile 222 als Mehrwegventile 246 ausgebildet. Die Figuren 2a) und 2b) zeigen hierzu exemplarisch ein 3-Wegeventil als erstes Ventil 224 und zweites Ventil 226. Eine alternative Ausführungsform eines Mehrwegventils wird im Folgenden anhand der Figuren 3a) und 3b) beschrieben.

Die zumindest eine erste und zweite Prozessiereinheit 30 und 32 können baugleich ausgebildet sein oder verschieden sein, um unterschiedliche Prozessarten durchführen zu können. Vorzugsweise sind die ersten Prozessiereinheiten 30 in der ersten Prozessiereinheitengruppe 13 und die zweiten Prozessiereinheiten 32 der zweiten Prozessiereinheitengruppe 15 baugleich ausgebildet. Es ist jedoch auch denkbar, dass sich die ersten Prozessiereinheiten 30 sowie die zweiten Prozessiereinheiten 32 sich jeweils untereinander unterscheiden. Beispielsweise kann die zumindest eine erste Prozessiereinheit 30 als Tiefenfilter ausgebildet sein, während die zumindest eine zweite Prozessiereinheit 32 als Sterilfilter ausgebildet sein kann.

**Fig. 2c****)** zeigt das Prozessiersystem 100 aus Fig. 2b), in dem die einzelnen Prozessiereinheiten 10 und die Adapterplatte 200 mit Hilfe von Abschlusshalterungen 34 zusammengehalten werden (Mehrwegventile 246 hier nicht gezeigt). Mit anderen Worten befindet am Eintrittspunkt, an dem der Fluidstrom 14 in das Prozessiersystem 100 eintritt, und am Austrittspunkt, an dem der Fluidstrom 14 aus dem Prozessiersystem 100 austritt, jeweils eine Abschlusshalterung 34. Diese kann vorzugsweise als Abschlussplatte ausgebildet sein, so dass die einzelnen Prozessiereinheiten 10 sandwichartig zwischen diesen Abschlussplatten gehalten werden. Ein- bzw. Auslässe können Teil der Abschlusshalterung 34 sein. Insbesondere kann aber zwischen einer Abschlusshalterung 34 und der jeweils angrenzenden Prozessiereinheit 10 eine weitere Abschlussplatte mit entsprechenden Anschlüssen eingebracht werden. Vorzugsweise wird der Fluidstrom 14 mit Hilfe einer Zuflusspumpe 35 in das Prozessiersystem 100 gepumpt. Figur 2c) zeigt eine Zuflusspumpe 35, welche bezüglich der Strömungsrichtung vor der Abschlusshalterung 34 angeordnet ist. Vorteilhafterweise kann sich die Zuflusspumpe 35 auch in der Adapterplatte 200 befinden, welche zwischen der Abschlusshalterung und der angrenzenden Prozessiereinheit 10 befindet. Hierdurch kann eine platzsparende Bauweise erzielt werden.

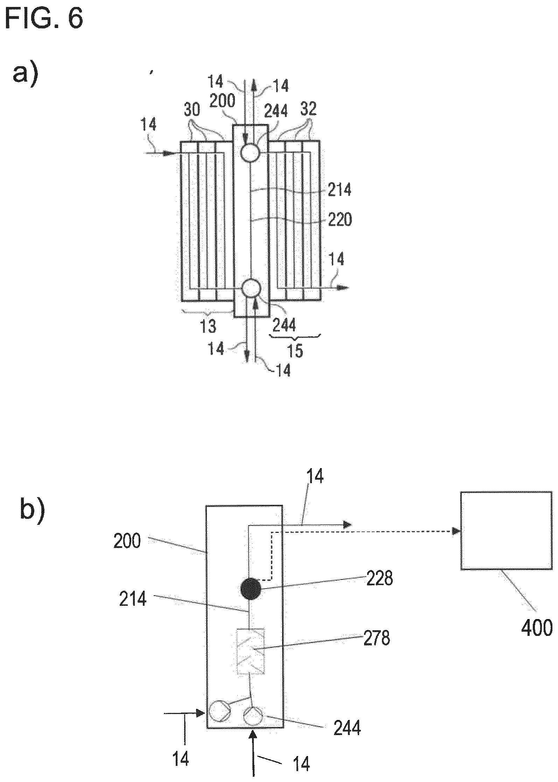

Obwohl alle Prozessiersysteme 100, welche in den Figuren 2a) bis 2c) gezeigt werden, Adapterplatten 200 umfassen, welche zwischen zwei Prozessiereinheiten 10 angeordnet sind, um diese fluidisch zu verbinden, ist diese Anordnung nicht zwingend erforderlich. Die Adapterplatte 200 kann zumindest einer Prozessiereinheit 10 lediglich vorgeschaltet sein, um ein Fluid der zumindest einen Prozessiereinheit 10 zuzuführen. Insbesondere kann die Adapterplatte 200 zwischen der Abschlusshalterung 34 und einer Prozessiereinheit 100 angeordnet sein. Dies ist beispielsweise dann vorteilhaft, wenn die nachgeschaltete Prozessiereinheit 10 eine Einheit zur Membranchromatographie ist. Üblicherweise werden im Rahmen der Chromatographie nacheinander verschiedene Medien auf das Filtermedium 16 gegeben. (Die Schritte Sanistieren/Konditionieren/Spülen/Äquilibrieren können beispielsweise vor dem Beladen mit der eigentlich zu reinigen Proteinlösung durchgeführt werden. Daher können die verschiedenen Eingänge an der Adapterplatte 200 dazu genutzt werden, um die einzelnen Medien der Prozessiereinheit 10 zuzuführen. Nach dem Beladen mit dem zu reinigenden Medium können anschließend ein Waschschritt und eine Elution erfolgen. Auch hier sind separate Eingänge und/oder Ausgänge an der Adapterplatte 200 hilfreich, wie dies beispielsweise bezüglich der Figur 6a) beschrieben wird. An einem derartigen Eingang und/oder Ausgang kann/können hierzu jeweils ein Umlenkelement vorgesehen sein, mit Hilfe dem zwischen Eingängen bzw. Ausgängen "geschaltet" werden kann).

Weiterhin weist das erfindungsgemäße Prozessiersystem 100 zumindest einen Sensor auf, der in zumindest einer Prozessiereinheit 10 und/oder zumindest einer Adapterplatte 200 eingebettet ist. Figur 2c) zeigt für die Ausführungsform der Figuren 2a) bis 2c) exemplarisch einen Adapterplatten-Sensor 228, welcher in der Adapterplatten 200 eingebettet ist, und einen Prozessiereinheiten-Sensor 36, welcher in der Prozessiereinheit 10, welche in Strömungsrichtung nach der Adapterplatte 200 angeordnet ist, eingebettet ist.

Insbesondere ist der zumindest eine Adapterplatten-Sensor 228 derart in die Adapterplatte 200 eingebettet, dass der Adapterplatten-Sensor 228 zumindest teilweise in den Adapterkanal 214 hineinragt, um mit dem Fluidstrom 14 in Kontakt zu treten und die gewünschten Messungen an dem Fluid vornehmen zu können.

Der Adapterplatten-Sensor 228 kann derart ausgebildet sein, dass beispielsweise der Druck, der Volumenstrom, der UV-Wert, der pH-Wert, die Trübung und/oder die Viskosität des Fluidstroms 14 gemessen werden kann. Die gemessenen Werte können dazu verwendet werden, den Filtrationsvorgang wiederum zu steuern bzw. zu regeln. Hierzu werden die gemessenen Werte an das externe Steuergerät 400 übermittelt. Der Adapterplatten-Sensor 228 kann als kostengünstiges Einweg-Bauteil ausgebildet sein, so dass der Adapterplatten-Sensor 228 zusammen mit der Adapterplatte 200 nach deren Verwendung entsorgt werden kann. Insbesondere kann der Adapterplatten-Sensor 228 bereits werksseitig in die Adapterplatte 200 integriert werden. Die Adapterplatte 200 wird dann bereits mit einem kalibrierten und sterilisierten Adapterplatten-Sensor 228 ausgeliefert.

Die gezeigte Adapterplatte 200 koppelt zwei Prozessiereinheiten 10 hier seriell. Es ist jedoch ebenfalls möglich, Adapterplatten-Sensoren 228 bei einer parallelen Verschaltung zweier Prozessiereinheiten 10 zu verwenden. Hierbei ist es jedoch vorteilhaft, dass der oder die Adapterplatten-Sensor(en) 228 in oder an dem ersten und/oder zweiten Kanalbereich 216 und 218 angeordnet ist/sind, um einen Kontakt zum Fluidstrom 14 herstellen zu können. Sind zwei Prozessiereinheiten 10 seriell verschaltet, kann der Adapterplatten-Sensor 228 auch in bzw. an dem Verbindungskanalbereich 220 angeordnet sein.

Weiterhin kann zumindest ein Adapterplatten-Sensor 228 an zumindest einem Umlenkelement in der zumindest einen Adapterplatte 200 vorgesehen sein. Dieser ist dazu ausgelegt, die Stellung des Umlenkelements zu detektieren, und diesen Zustand an das externe Steuergerät 400 zu übertragen.

Der zumindest eine Prozessiereinheiten-Sensor 36 kann zumindest einen Parameter des Fluidstroms 14 in der entsprechenden Prozessiereinheit 10 erfassen, wie dies bereits bezüglich des Adapterplatten-Sensors 228 beschrieben wurde. Insbesondere ist der Prozessiereinheiten-Sensor 36 derart in die Prozessiereinheit 10 eingebettet, dass dieser zumindest teilweise in Kontakt mit dem Fluidstrom 14 gelangt, um entsprechende Messungen vornehmen zu können. Die Messwerte können ebenfalls an das externe Steuergerät 400 übertragen werden.

Der Prozessiereinheiten-Sensor 36 kann an jeder beliebigen Position in der Prozessiereinheit 10 angeordnet sein, um die gewünschte Messung vorzunehmen. Beispielsweise kann der Prozessiereinheiten-Sensor 36 auf der Filtratseite 18 der Prozessiereinheit 10 angeordnet sein, um vorteilhaft das Filtrationsergebnis einer Prozessiereinheit 10 zu bestimmen bzw. zu überwachen. Insbesondere ist auch der Prozessiereinheiten-Sensor 36 vorzugsweise als kostengünstiges Einweg-Bauteil ausgebildet, so dass der Prozessiereinheiten-Sensor 36 nach seiner Verwendung zusammen mit der Prozessiereinheit 10 entsorgt werden kann.

Wie in Figur 2c) gezeigt, befindet sich vorzugsweise zumindest ein Prozessiereinheiten-Sensor 36 in der Prozessiereinheit 10, welche der Adapterplatte 200 nachgeschaltet ist. Dieser kann beispielsweise überwachen, mit welchem Druck der Fluidstrom 14 auf das Filtermedium 16 trifft. Prozessiereinheiten-Sensoren 36 können jedoch auch in jeder anderen der übrigen Prozessiereinheiten 10 des Prozessiersystems 100 vorgesehen sein.

**Figur 3a****)** zeigt eine Schnittansicht durch eine Adapterplatte 200. Wie in Figur 3a) gezeigt, ist die Adapterplatte 200 vorzugsweise zweiteilig ausgebildet und umfasst eine Zulaufplatte 230 und eine Ablaufplatte (hier nicht gezeigt). Als Zulaufplatte 230 wird die Platte der Adapterplatte 200 bezeichnet, in der die zumindest eine Zutrittsöffnung angeordnet ist. Als Ablaufplatte wird die Platte der Adapterplatte 200 bezeichnet, in der die zumindest eine Austrittsöffnung angeordnet ist. Die Form und/oder Größe der Zulaufplatte 230 und Ablaufplatte können identisch sein. In dem ersten und/oder zweiten Kanalbereich 216 und 218 befindet sich jeweils ein Mehrwegventil 246, das als Umlenkelement dient. Das Mehrwegventil 246 umfasst ein Ventilrohr 248, das sich jeweils zumindest teilweise in dem ersten und zweiten Kanalbereich 216 und 218 erstreckt und dort verlagerbar (insbesondere rotierbar bzw. drehbar) gelagert ist. Eine Verlagerung (insbesondere Rotation) kann mit Hilfe eines Umlenkaktuators 257, erfolgen. Der Umlenkaktuator 257 kann hierbei Befehle zur Verlagerung von dem externen Steuergerät 400 erhalten und diese in eine mechanische Verlagerung des Mehrwegventils 246 umsetzen. Der Umlenkaktuator 257 kann insbesondere an einem Verlagerungsgriff 254 angeordnet sein, der aus der Adapterplatte 200 ragt (siehe Figuren 3a) und 3b)). Wie in den Figuren 3a) und 3b) gezeigt, kann zumindest ein Adapterplatten-Sensor 228 an zumindest einem der Mehrwegventile 246 angeordnet sein, um die Stellung des Mehrwegventils 246 zu detektieren und diese Information an das externe Steuergerät 400 zu übertragen. Insbesondere kann der Adapterplatten-Sensor 228 an dem Verlagerungsgriff 254 des Mehrwegventils 246 angeordnet sein.

In einer Mantelfläche 250 des Ventilrohrs 248 sind zumindest zwei Ventilöffnungen 252 ausgebildet. Diese sind in Bezug auf die Verlagerungsrichtung (insbesondere Rotationsrichtung) derart voneinander versetzt angeordnet, dass in einer ersten Stellung des Ventilrohrs 248 ein erster Fluidstrom bzw. ein zweiter Fluidstrom ermöglicht wird. Das heißt, dass beispielsweise der erste Fluidstrom über die erste Zutrittsöffnung 202 der Adapterplatte 200 durch eine erste Ventilöffnung 252 die zumindest teilweise mit der ersten Zutrittsöffnung 202 überlappt, in das Ventilrohr 248 eintritt.

Die zweite Ventilöffnung 252 überlappt zumindest teilweise die erste Austrittsöffnung 210, so dass der erste Fluidstrom über die erste Austrittsöffnung 210 aus der Adapterplatte 200 strömen kann. Dies gilt in entsprechender Weise für den zweiten Fluidstrom und der zweiten Zutritts- und Austrittsöffnung 206 und 212. Ein Fluidstrom 14 durch den Verbindungskanalbereich 220 wird aufgrund der Stellung der Ventilöffnungen 252 in den jeweiligen Kanalbereichen blockiert. Da der erste und zweite Fluidstrom durch die Mehrwegventile 246 jedoch gestattet wird, sind die erste und zweite Prozessiereinheit 30 und 32, die jeweils direkt mit der Adapterplatte 200 gekoppelt sind, parallel miteinander verschaltet.

Vorzugsweise weisen die Ventilrohre 248 jedoch zumindest drei Ventilöffnungen 252 auf, wie in den Figuren 3a) und 3b) gezeigt. Diese sind derart angeordnet, dass in einer ersten Ausrichtung (insbesondere Rotationsstellung) des Ventilrohrs 248 zumindest zwei Ventilöffnungen 252 derart ausgerichtet sind, dass die oben beschriebenen ersten und zweiten Fluidströme gestattet werden und so die erste und zweite Prozessiereinheit 30 und 32, die jeweils direkt mit der Adapterplatte gekoppelt sind, parallel verschaltet sind. Ein Fluidstrom 14 durch den Verbindungskanalbereich 220 wird jedoch unterbunden, da keine Ventilöffnung 252 zumindest teilweise mit dem Verbindungskanalbereich 220 überlappt.

Aufgrund der Anordnung der zumindest drei Ventilöffnungen 252 sind in einer zweiten Ausrichtung (insbesondere Rotationsstellung) des jeweiligen Ventilrohrs 248 jedoch entweder der Durchgang zu einer Zutrittsöffnung oder zu einer Austrittsöffnung blockiert, während der Zugang zu dem Verbindungskanalbereich 220 gestattet wird. Dies ermöglicht eine serielle Verschaltung der ersten und zweiten Prozessiereinheit 30 und 32.

Mit anderen Worten sind in einer seriellen Verschaltung die Mehrwegventile 246 derart auszurichten, dass ein Fluidstrom 14 über die erste Zutrittsöffnung 202 der Adapterplatte 200 und über eine Ventilöffnung 252, die zumindest teilweise mit der ersten Zutrittsöffnung 202 überlappt, in das erste Ventilrohr 248 eintreten kann. Über eine Ventilöffnung 252, die zumindest teilweise mit dem Verbindungskanalbereich 220 überlappt, kann der Fluidstrom 14 in den Verbindungskanalbereich 220 strömen. Der Durchgang zu der ersten Austrittsöffnung 210 wird durch das Ventilrohr 248 blockiert, da keine Ventilöffnung 252 mit der ersten Austrittsöffnung 210 überlappt. Anschließend kann der Fluidstrom 14 über eine Ventilöffnung 252 des zweiten Ventilrohrs 248, die zumindest teilweise mit dem Verbindungskanalbereich 220 überlappt, in das zweite Ventilrohr 248 einströmen. Über eine Ventilöffnung 252 in dem zweiten Ventilrohr 248, die zumindest teilweise mit der zweiten Austrittsöffnung 212 der Adapterplatte 200 überlappt, kann der Fluidstrom 14 aus der Adapterplatte 200 ausströmen. Der Durchgang zu der zweiten Zutrittsöffnung 206 wird durch das Ventilrohr 248 blockiert, da keine Ventilöffnung 252 mit der zweiten Zutrittsöffnung 206 überlappt.

Somit kann in einfacher Weise und ohne konstruktive Umbaumaßnahmen zwischen einer seriellen und parallelen Verschaltung zweier Prozessiereinheiten 10 oder Prozessiereinheitengruppen 11 gewechselt werden. Insbesondere ist die Adapterplatte 200 als kompaktes Bauelement ausgelegt, so dass das Prozessiersystem 100 einen geringen Platzbedarf aufweist. Die Umlenkventile 222 können als kostengünstige Einweg-Bauteile ausgelegt sein. Mögliche Anwendungen können sein: Umschaltung für Tangentialstromfiltration von Standardbetrieb zu SinglePass, Umschaltung zwischen parallel und seriell verschalteten Chromatographieeinheiten beispielsweise zur seriellen Kombination von unterschiedlichen Chromatographiemedien oder zur Verbesserung der Kapazitätsausnutzung.

Die Figuren 3a) und b) beschreiben exemplarisch eine Ausführungsform eines rotierbaren Umlenkelements. Die Einstellung des Umlenkelements kann dabei über Steuersignale des externen Steuergeräts 400 erfolgen. Es wird darauf hingewiesen, dass dies jedoch für jede andere Art eines Umlenkelements erfolgen kann (siehe z.B. das oben beschriebene 3-Wege-Ventil in den Figuren 2a) bis 2c)).

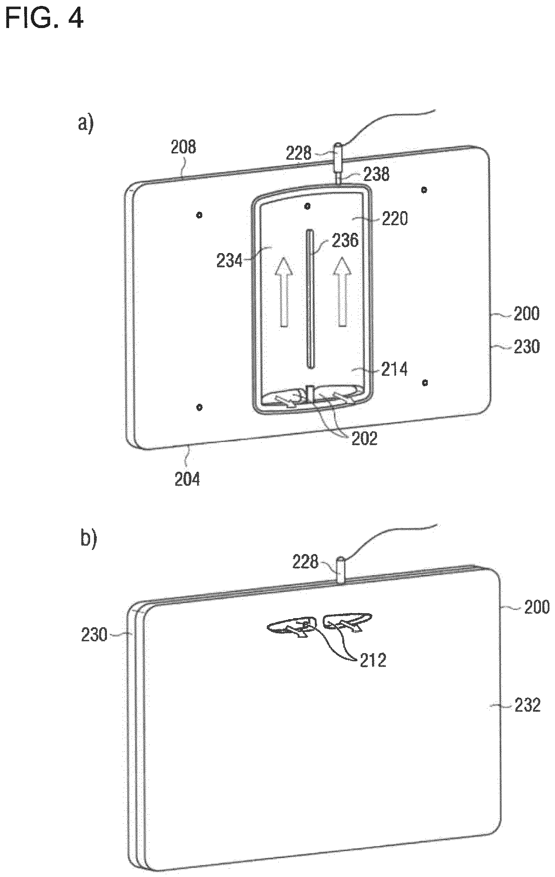

**Figuren 4a) und 4b****)** zeigen eine bevorzugte Ausführungsform einer Integration zumindest eines Adapterplatten-Sensor 228 in eine Adapterplatte 200. Wie in Figur 4b) gezeigt, ist die Adapterplatte 200 ebenfalls vorzugsweise zweiteilig bzw. mehrteilig ausgebildet. Die Zulaufplatte 230 umfasst die zumindest eine erste Zutrittsöffnung 202, vorzugsweise am unteren Ende 204 der Adapterplatte 200. Wie in Figur 4a) gezeigt, umfasst die Zulaufplatte 230 zwei erste Zutrittsöffnungen 202. Sie weist jedoch keine zweite(n) Zutrittsöffnung(en) 206 auf. Da die gezeigte Adapterplatte 200 ausschließlich eine serielle Verschaltung zweier Prozessiereinheiten 10 ermöglichen soll, sind diese jedoch auch nicht zwingend erforderlich. Für eine parallele Verschaltung könnte zumindest eine zweite Zutrittsöffnung 206, vorzugsweise am oberen Ende 208 der Adapterplatte 200, in der Zulaufplatte 230 ausgebildet sein.

Wie ferner in Figur 4b) gezeigt, umfasst die Ablaufplatte 232 zumindest eine zweite Austrittsöffnung 212. Im konkreten Fall der Figur 4b) weist die Ablaufplatte 232 zwei zweite Austrittsöffnungen 212 auf. Sollte eine parallele Verschaltung zweier Prozessiereinheiten 10 gewünscht sein, kann die Ablaufplatte 232 zusätzlich zumindest eine erste Austrittsöffnung 210 aufweisen. Die Zu- und Ablaufplatte 230 und 232 können miteinander verschraubt und/oder verklebt und/oder verschweißt und/oder verklickt sein.

In der Zu- und/oder Ablaufplatte 230 und 232 kann eine entsprechende Kanalvertiefung 234 ausgebildet sein. Sind die Zu- und Ablaufplatte 230 und 232 zusammengesetzt, ist so ein Adapterkanal 214 ausgebildet, der es ermöglicht, dass die Adapterplatte 200 von einem Fluidstrom 14 durchströmbar ist. Vorzugsweise ist in dem Verbindungskanalbereich 220 des Adapterkanals 214 zumindest ein Strömungssteg 236 ausgebildet, der sich vorzugsweise im Wesentlichen in Strömungsrichtung des Fluidstroms 14 erstreckt und dazu beiträgt, den Fluidstrom 14 in verbesserter Weise zu lenken. Der Adapterplatten-Sensor 228 kann in die Zu- und/oder Ablaufplatte 230 und 232 integriert sein und in den Adapterkanal 214 ragen, so dass ein Messelement des Adapterplatten-Sensors 228 mit dem Fluidstrom 14 in Kontakt treten kann. Vorzugsweise umfasst der Adapterkanal 214 zumindest bereichsweise einen Verbindungskanal 238 zum Adapterplatten-Sensor 228. Dieser Verbindungskanal 238 kann eine zumindest bereichsweise Vertiefung in dem Adapterkanal 214 sein, in die der Sensor 228 zumindest teilweise hineinragt.

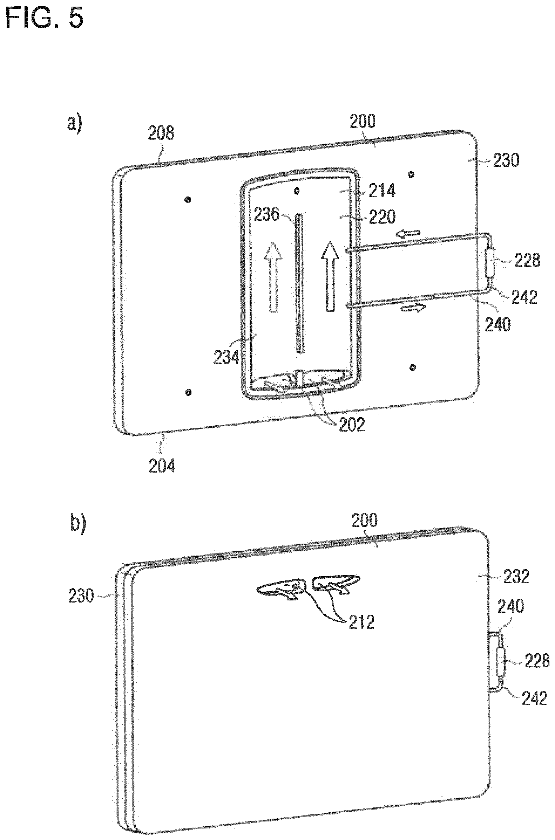

**Figuren 5a) und 5b****)** zeigen die Adapterplatte 200 aus den Figuren 4a) und 4b), jedoch ist in dieser Ausführungsform der Adapterplatten-Sensor 228 in bzw. an einer Hilfsabzweigung 240. Bei der Hilfsabzweigung 240 handelt es sich um eine Abzweigung aus dem Adapterkanal 214, in den zumindest ein Teil des Fluidstroms 14 abgezweigt wird. Dieser Teil des Fluidstroms 14 strömt zumindest für eine bestimmte Zeit aus dem Adapterkanal 214, um vorzugsweise später wieder in den Adapterkanal 214 zurückgeführt zu werden.

Die Hilfsabzweigung 240 kann dazu genutzt werden, kleine Mengen des Fluidstroms 14 für entsprechende Messungen abzuzweigen. Die Hilfsabzweigung 240 kann mit Hilfe eines separaten Kanalelements 242 ausgebildet sein, das zumindest teilweise in die Adapterplatte 200 integriert ist. Wie in den Figuren 5a) und 5b) gezeigt, kann ein Teil der Hilfsabzweigung 240 aus der Adapterplatte 200 herausragen. Insbesondere kann hierdurch auch der Adapterplatten-Sensor 228 außerhalb der Adapterplatte 200 angeordnet sein. In dieser Anordnung wird der Adapterplatten-Sensor 228 jedoch auch als in die Adapterplatte 200 "eingebettet" angesehen.

**Figur 6a****)** zeigt ein Prozessiersystem 100 mit einer ersten und zweiten Prozessiereinheitengruppe 13 und 15, die mittels einer Adapterplatte 200 gekoppelt sind. Die verknüpften Prozessiereinheitengruppen 11 sind in der gezeigten Ausführungsform seriell durch die Adapterplatte 200 verschaltet, es ist jedoch ebenfalls möglich, die beiden Prozessiereinheitengruppen 11 parallel gemäß der übrigen beschriebenen Ausführungsformen zu verschalten. Wie oben beschrieben, kann die Adapterplatte 200 auch nur als vorgeschaltete Adapterplatte 200 ausgebildet sein.

In der Adapterplatte 200 kann gemäß Figur 6a) zumindest ein Hilfsausgang- und/oder -zugang 244 ausgebildet sein. Über einen Hilfsausgang kann zumindest ein Teil des Fluidstroms 14 aus dem Adapterkanal 214 entnommen werden bzw. herausströmen. Über einen Hilfszugang kann der entnommene Fluidstrom 14 wieder zurück in den Adapterkanal 214 strömen oder Zugriff zu dem Adapterkanal 214 gewährleistet werden. Die Verwendung mindestens eines Hilfsausgangs bzw. -zugangs ermöglicht beispielsweise die externe Zuschaltung einer Pumpe, einen Integritätstest an unterschiedlichen Prozessiereinheiten 10 des Prozessiersystems 100, die Abfuhr des Fluids aus der ersten oder weiteren vorgeschalteten Prozessiereinheiten 30 zur zwischenzeitlichen Pufferung oder zur weiteren Prozessierung, eine Entlüftung und/oder eine Probeentnahme. Insbesondere kann zumindest ein Diafiltrationsmedium und/oder andere Reagenzien über einen Hilfszugang zugegeben werden. Figur 6a) zeigt eine Adapterplatte 200 mit zwei Hilfsaus- und -zugängen 244. Mögliche Anschlüsse an einem Hilfsaus- und Hilfszugang 244 könnten Triclamps oder Sterilkonnektoren sein. Um das Fluid an dem Hilfsaus- bzw. zugang 244 zuzuführen bzw. abzuführen, kann entsprechend an dem Hilfsaus- bzw. zugang 244 eine Hilfspumpe oder ein Hilfsventil vorgesehen sein.

Figur 6b) zeigt eine Ausführungsform einer Adapterplatte 200 mit zumindest einem Hilfszugang 244, der dazu verwendet werden kann, ein weiteres Fluid zur Verdünnung dem Fluids, welches sich bereits in der Adapterplatte 200 befindet, zuzufügen. Eine Vermischung des weiteren Fluids mit dem bereits in der Adapterplatte 200 enthaltenen Fluid kann über einen statischen Mischer 278 erfolgen. Eine beispielshafte Anwendung, in der eine Verdünnung notwendig ist, ist die Notwendigkeit einer Senkung der Salzkonzentration in dem Fluid während eines Teilschritts des Antibody-Polishing-Verfahrens.

Als "statischer Mischer" 278 wird eine Vorrichtung zum Mischen von Fluiden verstanden, in der alleine die Strömungsbewegung die Vermischung bewirkt. Um eine vermischende Strömungsbewegung zu erzielen, werden strömungsbeeinflussende Elemente in dem Adapterkanal 214 bereitgestellt. Derartige Elemente können aneinandergereite schrauben-, lamellen- oder gitterförmige Elemente sein. Die zu vermischenden Fluide werden gemeinsam und im gewünschten Mischungsverhältnis dem Mischer 278 zugeführt. Die Elemente teilen den Stoffstrom, verdrehen die Ströme und führen sie wieder zusammen, so dass die gewünschte Vermischung erreicht wird.

Im konkreten Fall der vorliegenden Adapterplatte 200 wird der Adapterplatte 200 ein Fluidstrom 14 zugeführt. Dieser kann beispielsweise einer vorgeschalteten Prozessiereinheit 10 entstammen. Um diesen Fluidstrom 14 nun in der Adapterplatte 200 zu verdünnen, kann über einen Hilfszugang 244 ein weiterer Fluidstrom 14 der Adapterplatte 200 zugeführt werden. Diese beiden Fluidströme 14 werden anschließend mittels des statischen Mischers 278 vermischt.

Vorzugsweise umfasst die Adapterplatte 200 zumindest einen Adapterplatten-Sensor 228, der dazu ausgelegt ist, die Verdünnung zu überwachen. Hierzu ist der Adapterplatten-Sensor 228 vorzugsweise in Strömungsrichtung nach dem statischen Mischer 278 angeordnet. Die detektierten Werte des Adapterplatten-Sensor 228 können dann anschließend an das externe Steuergerät 400 übertragen werden. Dieses kann auf Basis der erhaltenen Messdaten beispielsweise eine Hilfspumpe an dem Hilfszugang 244 regeln, um die zuzuführende Menge des Verdünnungsfluids zu regeln.

Sollte der Verdünnungsgrad nicht den erforderlichen Wert aufweisen, könnte der Fluidstrom 14 vorzugsweise auch über einen Hilfsausgang 244 abgeführt werden. Der abgeführte Fluidstrom könnte anschließend wieder rückgeführt werden, um weiter verdünnt zu werden oder komplett verworfen werden. Nur ein Fluidstrom 14 mit der gewünschten Verdünnung kann anschließend der angeschlossenen Prozessiereinheit 10 zugeführt werden.

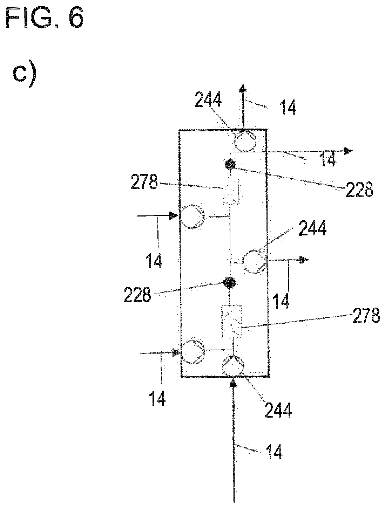

Figur 6c) zeigt eine weitere Ausführungsform einer Adapterplatte 200 mit zumindest einem Hilfsaus- bzw. zugang 244. Hier kann die Adapterplatte 200 insbesondere zur Virusinaktivierung verwendet werden.

Der Aufbau ist grundsätzlich ähnlich zur Figur 6b). Statt einer Verdünnungslösung wird hier jedoch ein Fluid zur pH-Senkung über einen Hilfszugang 244 zugegeben. Mit Hilfe des statischen Mischers 278 wird der pH-Senker mit dem Fluidstrom 14, welcher die Adapterplatte 200 durchströmt, vermischt.

Ähnlich zu der Figur 6b), kann sich nach dem statischen Mischer 278 zumindest ein Adapterplatten-Sensor 228 befinden. Dieser detektiert hier vorzugsweise den pH-Wert des Fluidstroms 14 nach dem statischen Mischer 278. Die Messdaten werden an das externe Steuergerät 400 übertragen. Ist der pH-Wert zu hoch, kann der Fluidstrom 14 über einen Hilfsausgang 244 aus der Adapterplatte 200 abgeführt werden. Entweder wird der abgeführte Fluidstrom 14 in die Adapterplatte 200 rückgeführt und der pH-Wert weiter gesenkt oder das Fluid wird verworfen. Das externe Steuergerät 400 kann insbesondere auf Basis der erhaltenen Messdaten beispielsweise eine Hilfspumpe an dem Hilfszugang 244 regeln, um die zuzuführende Menge des pH-Senkers zu regeln.

Hat das Fluid einen geeigneten pH-Wert, kann das Fluid in der Adapterplatte 200 weiterströmen. Um den pH-Wert wieder zu erhöhen, befindet sich hier anschließend ein weiterer statischer Mischer 278. Über einen weiteren Hilfszugang 244 wird ein Fluid zur pH-Wert Erhöhung zugegen und mit dem Fluidstrom 14 mit Hilfe des weiteren statischen Mischers 278 in der Adapterplatte 200 vermischt. Zumindest ein weiterer Adapterplatten-Sensor 228 nach dem weiteren statischen Mischer 278 überwacht vorzugsweise wiederum den erreichten pH-Wert des Fluids und übermittelt die Messdaten an das externe Steuergerät 400. Auch hier kann wie bereits bei dem vorherigen statischen Mischer 278 eine Rückführung oder ein Verwerf des fehlerhaften Fluids über einen weiteren Hilfsausgang 244 erfolgen. Das externe Steuergerät 400 kann insbesondere auf Basis der erhaltenen Messdaten beispielsweise eine Hilfspumpe an dem Hilfszugang 244 regeln, um die zuzuführende Menge des pH-Erhöhers zu regeln.

Um eine vollständige Virusinaktivierung zu erzielen, kann der Adapterkanal 214 zwischen den beiden statischen Mischern 278 derart ausgebildet sein, dass das Fluid eine ausreichende Zeit mit dem gesenkten pH-Wert verbleibt. Vorzugsweise ist diese Teilstrecke mäanderförmig ausgebildet, so dass das Fluid vorzugsweise 30 min in dem gesenkten pH-Wert verbleibt.

Es wird darauf hingewiesen, dass der Aufbau des Adapterkanals 214 in den Figuren 6a) bis 6c) hier exemplarisch gezeigt wird. Der Adapterkanal 214 kann entsprechend jeder vorangehend beschriebenen Ausführungsformen aufgebaut sein. Insbesondere wird anhand der Figuren 6a) bis 6c) ein Aufbau beschrieben, bei dem alle Schritte bzw. alle Komponenten einer Virusinaktivierung in einer Adapterplatte 200 vorgesehen sind. Es können jedoch auch mehrere Adapterplatten 200 hintereinander geschaltet werden bzw. miteinander gekoppelt werden und die einzelnen Komponenten auf diese Adapterplatten 200 verteilt sein. Das heißt, die Virusinaktivierung ist dann erfolgt, wenn der Fluidstrom 14 die einzelnen Adapterplatten 200 mit den verschiedenen Komponenten zur Virusinaktivierung durchströmt hat.

Hilfspumpen können in Adapterplatte 200 angeordnet sein, wie in den Figuren 6b) und 6c) gezeigt wird. Eine Hilfspumpe kann jedoch auch außerhalb an der Adapterplatte 200 oder als externes Gerät angeordnet sein.

Weiterhin kann die Teilstrecke zwischen den beiden statischen Mischern 278, wie bezüglich Figur 6c) beschrieben wurde, auch zumindest teilweise außerhalb der Adapterplatte 200 verlaufen.

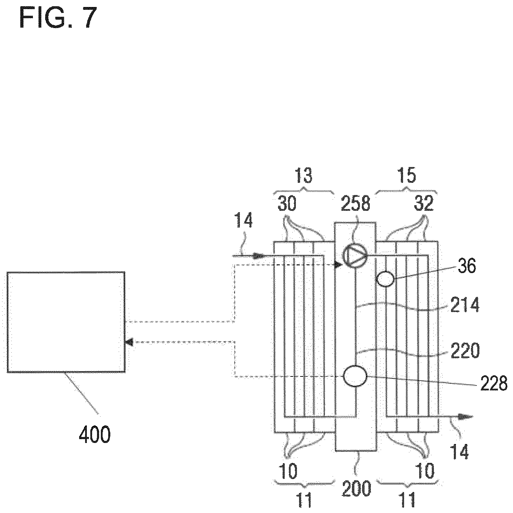

**Figur 7** zeigt ein Prozessiersystem 100 mit einer ersten und zweiten Prozessiereinheitengruppe 13 und 15. Die erste und zweite Prozessiereinheitengruppe 13 und 15 sind mittels einer Adapterplatte 200 gekoppelt. In bzw. an dem Adapterkanal 214 der Adapterplatte 200 ist zumindest eine Pumpe 258 angeordnet. Diese kann bei einer parallelen Verschaltung zweier Prozessiereinheitengruppen 11 genutzt werden, ist jedoch besonders in einer seriellen Verschaltung, wie in Figur 7 gezeigt, vorteilhaft da hierdurch der Druck gesteuert werden kann, mit das Fluid auf die in Strömungsrichtung folgende Prozessiereinheit 10 trifft. Insbesondere ein Flusswiderstand kann hierdurch überwunden werden, so dass eine hinreichende Filtrationsleistung in der zweiten Prozessiereinheitengruppe 15 erzielt werden kann.

Weiterhin kann die Pumpe 258 insbesondere als Verdrängerpumpe ausgebildet sein, um in der ersten Prozessiereinheitengruppe 13 einen Unterdruck zu initiieren und in der zweiten Prozessiereinheitengruppe 15 einen gewünschten Filtrationsdruck aufzubauen. Insbesondere kann die Pumpe 258 als kostengünstiges Einweg-Bauteil ausgebildet sein.

Die Pumpe 258 ist mit dem externen Steuergerät 400 gekoppelt. Über das externe Steuergerät 400 erhält die Pumpe 258 Steuerbefehle, die die Pumpe 258 aktivieren, deaktiveren oder steuern bzw. regeln können.

Die Steuerung bzw. Regelung der Pumpe 258 basiert vorzugsweise auf Messdaten zumindest eines Adapterplatten-Sensors 228 und/oder zumindest eines Prozessiereinheiten-Sensors 36, der/die zumindest einen Parameter des Fluidstroms 14 in dem Prozessiersystem 100 erfasst haben. Beispielsweise können Messdaten des zumindest einen Sensors von dem externen Steuergerät ausgelesen werden und dazu genutzt werden, die Pumpe 258 zu regeln, um den gewünschten Druck des Fluidstroms 14 zu erzielen. Hierzu können Grenzwerte in dem externen Steuergerät 400 festgelegt sein. Weicht der Druck des Fluidstroms 14 von den definierten Werten ab, kann das externe Steuergerät 400 die Einstellungen der Pumpe 258 adaptieren, so dass der Fluidstrom 14 den gewünschten Druck aufweist. Eine Adaptierung der Einstellungen an der Pumpe 258 können über einen Pumpen-Aktuator vorgenommen werden, der die Steuerbefehle des externen Steuergeräts 400 erhält.

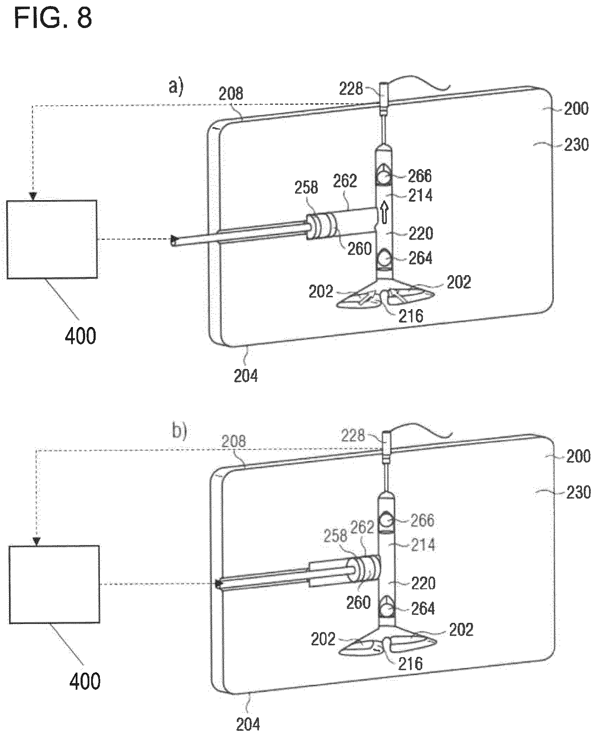

**Figuren 8a) und 8b****)** zeigen eine konkrete Ausführungsform einer Adapterplatte 200 mit einer Verdrängerpumpe, nämlich einer Kolbenpumpe. Die Pumpe 258 ist vorzugsweise in dem Verbindungskanalbereich 220 angeordnet und umfasst zumindest einen Kolben 260, welcher ausgelegt ist, eine Hub-Bewegung, also eine geradlinige (translatorische) Bewegung, auszuführen. Hierzu ist der Kolben 260 in einem Zylinder 262 gelagert. Zusätzlich weist die Pumpe 258 einen Zu- und einen Ablauf auf, die jeweils durch ein Ventil verschließbar sind.

Im Speziellen ist ein Abschnitt in dem Verbindungskanalbereich 220 in Flussrichtung durch ein Einlassventil 264 und durch ein Auslassventil 266 abgetrennt bzw. abgegrenzt. Über das Einlassventil 264 kann das Fluid in diesen Abschnitt strömen und über das Auslassventil 266 entweichen. Der Zylinder 262, in dem der Kolben 260 gelagert ist und in diesem eine translatorische Bewegung ausführen kann, ist derart angeordnet, dass der Zylinder 262 an dem Verbindungskanalbereich 220 angeordnet und mit diesem fluidisch verbunden ist. Insbesondere ist der Zylinder 262 zwischen dem Einlass- und Auslassventil 264 und 266 an dem Verbindungskanalbereich 220 angeordnet.

In einem ersten Takt, beim Ansaugen, führt der Kolben 260 eine Rückwärtsbewegung aus, also eine Bewegung weg von dem Verbindungskanalbereich 220. Das Einlassventil 264 öffnet sich und das zu fördernde Fluid kann in den Verbindungskanalbereich 220 bzw. den Zylinder 262 strömen. In einem zweiten Takt, bei der Förderbewegung schließt sich das Einlassventil 264 und der Kolben 260 bewegt sich in Richtung zu dem Verbindungskanalbereich 220. Es öffnet sich das Auslassventil 266 und das Fördermedium wird herausgedrückt.

Vorzugsweise ist zumindest ein Adapterplatten-Sensor 228 in dem Adapterkanal 214 angeordnet, um den Fluidstrom 14 zu überwachen. Hierzu kann der Adapterplatten-Sensor 228 vorzugsweise in Flussrichtung nach der Pumpe 258 angeordnet sein, um beispielsweise den Fluiddruck oder den Fluidfluss zu messen. Dieser Wert kann dazu verwendet werden, mittels des externen Steuergeräts 400 die Pumpe 258 zu regeln.

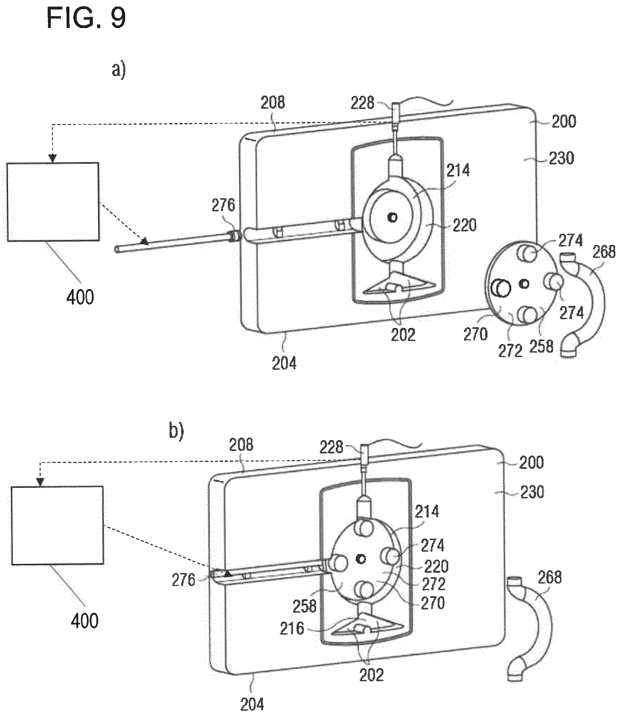

Eine weitere Möglichkeit einer Verdrängerpumpe wird anhand der **Figuren 9a) und 9b****)** gezeigt. Die in die Adapterplatte 200 integrierte Pumpe 258 ist eine Peristaltikpumpe. Diese ist vorzugsweise aus den bereits oben beschriebenen Gründen in dem Verbindungskanalbereich 220 angeordnet.

Eine Peristaltikpumpe, auch Schlauchpumpe genannt, ist eine Verdrängerpumpe, bei der das zu fördernde Fluid durch äußere mechanische Verformung eines Schlauches 268 durch diesen hindurchgedrückt wird. Der Schlauch 268 stellt in dem vorliegenden Fall somit einen Teil des Verbindungskanalbereichs 220 dar, durch den das Fluid in der Adapterplatte 200 strömt. Der Bereich, in dem der Schlauch 268 angeordnet ist, ist kreisförmig bzw. der Verbindungskanalbereich 220 weitet sich bogenförmig auf. In dem kreisförmigen Abschnitt des Verbindungskanalbereichs 220 ist ein Rotor 270 angeordnet, der dort drehbar gelagert ist.

Der Rotor 270 ist vorzugsweise als kreisrunde Platte ausgebildet. Auf einer Oberseite 272 des Rotors 270 ist/sind zumindest eine Rolle 274 und/oder ein Gleitschuh angeordnet. Der Schlauch 268 liegt zumindest bereichsweise an einer Mantelfläche des kreisförmigen Abschnitts des Verbindungskanalbereichs 220 an. Durch eine Rotation des Rotors 270 ist der Schlauch 268 von innen durch die Rollen 274 und/oder Gleitschuhe abklemmbar. Dies führt dazu, dass sich eine Abklemmstelle entlang des Schlauches 268 bewegt und dadurch das zu fördernde Fluid vorantreibt. Der Rotor 270 ist über einen Zahnradmechanismus 276, wie in den Figuren 9a) und 9b) gezeigt, drehbar. Zumindest ein Adapterplatten-Sensor 228 kann in der Adapterplatte 200 angeordnet sein, wie dies bezüglich vorangehender Ausführungsformen beschrieben wurde. Insbesondere kann der Zahnradmechanismus 276 von dem externen Steuergerät 400 über vorzugsweise einen Aktuator gesteuert werden. Die Steuerbefehle des externen Steuergeräts 400 basieren dabei zumindest teilweise auf Messwerten des zumindest einen Adapterplatten-Sensors 228 und/oder des zumindest einen Prozessiereinheiten-Sensors 36 in dem Prozessiersystem 100.

Weitere Anmerkungen zu den vorangehend beschriebenen Ausführungsformen:
In verschiedenen Ausführungsformen wird die Adapterplatte 200 zur Verknüpfung zweier Prozessiereinheitengruppen 11 beschrieben. Es wird jedoch darauf hingewiesen, dass die Beschreibungen ebenfalls entsprechend für Ausführungsformen gelten, in denen nur eine erste und/oder zweite Prozessiereinheit 30 und 32 vorgesehen sind.

In den beschriebenen Prozessiersystemen 100 wird jeweils eine Adapterplatte 200 gezeigt, die zwei Prozessiereinheitengruppen 11 koppelt. Es ist jedoch möglich, dass in einem Prozessiersystem 100 eine Vielzahl von Adapterplatten 200 enthalten ist. Prozessiereinheitengruppen 11, die durch eine Adapterplatte 200 gekoppelt sind, werden hier immer als erste und zweite Prozessiereinheitengruppe 13 und 15 benannt und die obige Beschreibung gilt entsprechend für jede Verknüpfung zweier Prozessiereinheitengruppen 11. Gleiches gilt ebenfalls für einzelne durch eine Adapterplatte 200 verknüpfte Prozessiereinheiten 10 oder einzelne Prozessiereinheiten 10, die mit einer Prozessiereinheitengruppe 11 verknüpft sind.

In den oben beschriebenen Ausführungsformen wurde eine Pumpe 258 in der Adapterplatte 200 als Mittel zur Druckregulierung des Fluidstroms 14 beschrieben. Alternativ oder ergänzend zur Pumpe 258 kann zumindest ein Ventil in dem Adapterkanal 214 vorgesehen sein. Das Ventil ist dazu ausgelegt, den Adapterkanal 214 zu verengen bzw. die Größe des Querschnitts des Adapterkanals 214 zu variieren und so den Druck des Fluidstroms 14 zu regulieren. Das Ventil kann durch das externe Steuergerät 400 gesteuert werden. Insbesondere basieren die Steuersignale auf gemessenen Druckparametern, die durch zumindest einen Adapterplatten-Sensor 228 und/oder Prozessiereinheiten-Sensor 36 gemessen wurden.

Die oben beschriebenen Ausführungsformen von Pumpen 258 in einer Adapterplatte 200 können alternativ als Pressluftpumpe ausgeführt sein, in welcher vorzugsweise zumindest eine Membran auf- und abbewegt wird, so dass das Fluid in Strömungsrichtung bewegt wird.

Eine Aktuation der oben vorangehend beschriebenen Ventile kann grundsätzlich elektrisch, mechanisch, pneumatisch oder hydraulisch erfolgen.

Weiterhin wird darauf hingewiesen, dass, obwohl die gezeigten Adapterplatten 200 zweiteilig ausgebildet sind, es ebenfalls denkbar ist, diese einteilig auszubilden.

In der obigen Beschreibung wird auf den biopharmazeutischen Einsatz der Prozessiersysteme 100 bzw. der Adapterplatten 200 abgezielt. Es ist jedoch ebenfalls möglich, das gezeigte Prinzip auf andere Prozesse zu übertragen, wie z.B. die Lebensmittelherstellung, chemische Produktionsprozesse, Getränkefiltration, Fraktionieren von Partikeln, Schmutzwasseraufbereitung und dgl.

Insbesondere wird darauf hingewiesen, dass die Adapterplatte 200 in den beschriebenen Ausführungsformen jeweils direkt bzw. ohne Zwischenschaltung weiterer Elemente mit einer zweiten Prozessiereinheit 32 gekoppelt ist. Es ist jedoch möglich, die Adapterplatte 200 mit der zweiten Prozessiereinheit 32 über eine kompatible Konnektierung zu verbinden. Diese ist vorzugsweise steril und/oder tropffrei ausgebildet. Zudem können auch Tanks oder andere Komponente zwischengeschaltet sein.

Die Adapterplatte 200 kann außerdem auch vor und/oder nach der letzten Prozessiereinheit 10 eines Prozessiersystems 100 angeordnet sein. Als eine oder mehrere Prozessiereinheiten 10 kann insbesondere eine Filterkassette "Sartoclear^{®} Depth Filter Cassette" und/oder eine Filterkassette der "Sartoclear^{®} DL-Serie" und/oder eine Filterkassette der "Sartoclear^{®} S-Serie" von Sartorius Stedim Biotech GmbH zum Einsatz kommen.

Die verschiedenen Ausführungsformen der Prozessiersysteme 100 bzw. der Adapterplatten 200 wurden getrennt anhand der einzelnen Figuren beschrieben. Es wird jedoch darauf hingewiesen, dass die einzelnen Ausführungsformen bzw. Teile der einzelnen Ausführungsformen miteinander kombinierbar sind. Um Wiederholungen zu vermeiden, wurden außerdem bezüglich der einzelnen Ausführungsformen bereits beschriebene Elemente nicht noch einmal beschrieben.

Im Folgenden wird nun die Signalübertragung in dem Prozessiersystem 100 beschrieben:
Wie bereits oben im Detail erläutert wurde, weist/weisen die zumindest eine Adapterplatte 200 und/oder die zumindest eine Prozessiereinheit 10 jeweils zumindest einen Sensor auf. Dieser misst zumindest einen Parameter des Fluidstroms 14 in dem Prozessiersystem 100. Der zumindest eine Sensor ist mit dem externen Steuergerät 400 entweder kabelgebunden oder kabellos gekoppelt, so dass Messwerte des zumindest einen Sensors an das externe Steuergerät 400 übertragen werden können.

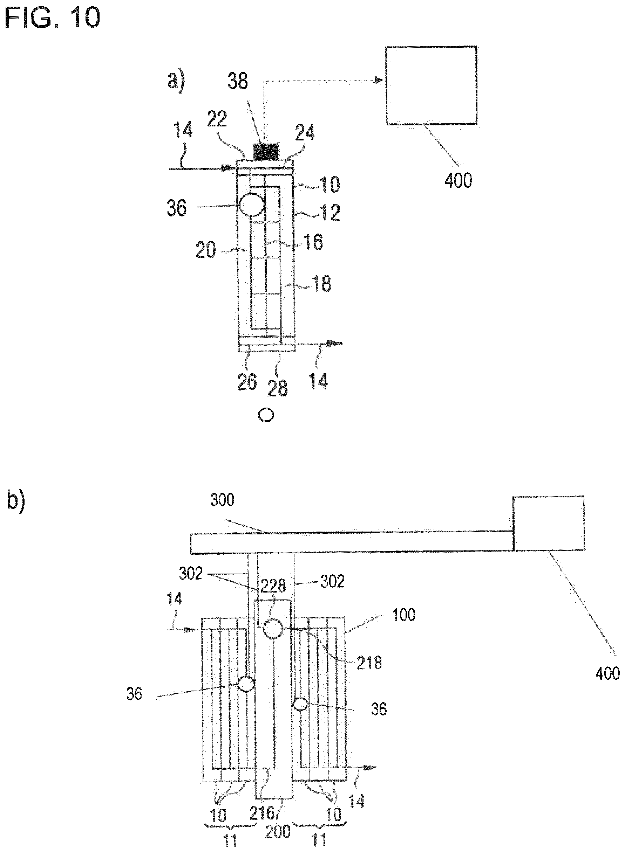

Figur 10a) zeigt eine Prozessiereinheit 10, in der ein Prozessiereinheiten-Sensor 36 vorgesehen bzw. eingebettet ist. Dieser misst beispielsweise den Druck des Fluidstroms 14 bevor das Fluid auf das Filtermedium 16 trifft. Hierdurch kann überwacht werden, ob der Fluidstrom 14 einen ausreichenden Druck aufweist, so dass eine zufriedenstellende Filterleistung erzielt werden kann.

Die Prozessiereinheit 10 weist zumindest einen Prozessiereinheiten-Transponder 38 auf, der in oder an der Prozessiereinheit 10 angeordnet ist. Figur 10a) zeigt beispielhaft einen Prozessiereinheiten-Transponder 38, der an der Prozessiereinheit 10 angeordnet ist. Der Prozessiereinheiten-Transponder 38 ist mit dem Prozessiereinheiten-Sensor 36 entweder mittels Kabel, kabellos oder optisch gekoppelt und erhält Messdaten von dem Prozessiereinheiten-Sensor 36. Insbesondere werden die Messdaten zusammen mit einer eindeutigen Adresse des Prozessiereinheiten-Sensors 36 übermittelt, so dass die Messdaten klar einem Sensor zuordenbar sind. Diese Daten werden von dem Prozessiereinheiten-Transponder 38 über Funk an das externe Steuergerät 400 übertragen. Die Übermittlung erfolgt vorzugsweise automatisch.

Der Prozessiereinheiten-Transponder 36 kann als passiver Transponder ausgebildet sein, vorzugsweise als "Radio Frequency Identification Device" (RFID), oder als aktiver Transponder mit eigener Energieversorgung. Hierzu weist der Prozessiereinheiten-Transponder 36 einen Akku auf oder ist an ein externes Stromnetz angeschlossen. Insbesondere kann der Prozessiereinheiten-Transponder 36 den Datenübertragungsstandard "Near Field Communication" (NFC) verwenden.

Die Datenübertragung kann vorzugsweise per WLAN, Bluetooth und/oder Mobilfunkstandard (z.B. LTE, 5G, GSM) erfolgen.

Weist die Prozessiereinheit 10 mehrere Prozessiereinheiten-Sensoren 36, können alle oder zumindest mehrere Prozessiereinheiten-Sensoren 36 ihre Messdaten über einen Prozessiereinheiten-Transponder 38 an das externe Steuergerät 400 übertragen. Alternativ kann der Prozessiereinheiten-Sensor 36 mit einem integrierten Transponder ausgestaltet sein, so dass jeder Prozessiereinheiten-Sensor 36 einen eigenen Transponder aufweist.

Die Verwendung von Transpondern zur kabellosen Datenübertragung von Sensordaten an das externe Steuergerät 400 wurde exemplarisch anhand einer Prozessiereinheit 10 erläutert. Weist das Prozessiersystem 100 mehrere Prozessiereinheiten 10 auf, die jeweils zumindest einen Prozessiereinheiten-Sensor 36 aufweisen, können diese oder zumindest Teile davon gemeinsam einen Prozessiereinheiten-Transponder 38 zur Datenübertragung nutzen. Der gemeinsam genutzte Prozessiereinheiten-Transponder 38 kann in oder an einer der Prozessiereinheiten 10 angeordnet sein oder als externe Einheit ausgebildet sein.

Es wird darauf hingewiesen, dass die Verwendung von zumindest einem Transponder zur kabellosen Datenübertragung hier exemplarisch für zumindest eine Prozessiereinheit beschrieben wurde. Die Verwendung von zumindest einem Transponder kann jedoch entsprechend auch für zumindest einen Adapterplatten-Sensor 228 in zumindest einer Adapterplatte 200 erfolgen. Alternativ besteht auch die Möglichkeit, dass zumindest ein Prozessiereinheiten-Sensor 36 und zumindest ein Adapterplatten-Sensor 228 gemeinsam einen Transponder nutzen. Dieser kann in oder an einer Prozessiereinheit 10, einer Adapterplatte 200 oder als externes Gerät angeordnet sein.

**Figur 10b****)** zeigt eine Ausführungsform eines Prozessiersystems 100 mit einer kabelgebundenen Datenübertragung von dem zumindest einen Sensor zu dem externen Steuergerät 400.

Im Speziellen zeigt Figur 10b) ein Prozessiersystem 100 mit zwei Prozessiereinheitengruppen 11, welche durch eine Adapterplatte 200 gekoppelt sind. Exemplarisch weisen zwei der Prozessiereinheiten 10 einen Prozessiereinheiten-Sensor 36 auf und die Adapterplatte 200 weist einen Adapterplatten-Sensor 228 auf. Wie bereits vorangehend beschrieben wurde, ist die Anzahl der Prozessiereinheiten 10 als auch die Anzahl der Adapterplatten 200 jedoch variabel. Weiterhin sind die Anzahl und der Ort der Sensoren in dem Prozessiersystem 100 entsprechend der vorangehenden Beschreibungen zu anderen Ausführungsformen variabel.

Die einzelnen Sensoren 228, 36 sind hier über ein Bussystem 300 mit dem externen Steuergerät 400 gekoppelt. Das Bussystem 300 umfasst zumindest eine Busleitung, welche vorzugsweise entlang der zumindest einen Prozessiereinheit 10 und der zumindest einen Adapterplatte 200 verläuft. Die einzelnen Sensoren 228, 36 des Prozessiersystems 100 sind mittels Kabel 302 mit der zumindest einen Busleitung gekoppelt. Jeder Sensor 228, 36 kann hierzu mit einer separaten Busleitung gekoppelt sein oder mehrere Sensoren 228, 36 nutzen dieselbe Bussleitung. Insbesondere können die Kabel 302 mehrerer Sensoren 228, 36, welche in einer Prozessiereinheit 10 oder einer Adapterplatte 200 eingebettet sind, zunächst in der Prozessiereinheit 10 oder in der Adapterplatte 200 zusammengeführt werden, so dass nur ein Kabel 302 aus der Prozessiereinheit 10 oder der Adapterplatte 200 herausgeführt wird und mit einer Busleitung zu koppeln ist. Die Messdaten der Sensoren 228, 36 werden entsprechend der kabellosen Datenübertragung, wie bezüglich der Figur 10a) beschrieben, übertragen und von dem externen Steuergerät 400 ausgewertet.

Die Busleitungen können vorzugsweise in zumindest einer Schiene angeordnet sein, die entlang der zumindest einen Prozessiereinheit 10 und der zumindest einen Adapterplatte 200 verläuft.

Die Sensoren 228, 36 können mit einem Akku ausgestattet sein, der die Sensoren 228, 36 während ihrer Betriebszeit mit der erforderlichen Energie versorgt. Alternative können die einzelnen Sensoren 228, 36 jedoch über eine externe Stromquelle mit Energie gespeist werden. Das zumindest eine Kabel, das zur Energieversorgung der Sensoren 228, 36 benötigt wird, kann hierzu ebenfalls in der oben beschriebenen Schiene verlaufen und anschließend mit den einzelnen Sensoren 228, 36 gekoppelt werden. Befinden sich mehrere Sensoren 228, 36 in einer Prozessiereinheit 10 oder Adapterplatte 200 ist es bevorzugt, dass jeweils ein Hauptenergieversorgungskabel aus der Prozessiereinheit 10 und der Adapterplatte 200 ragt, das dann mit einem Hauptkabel, welches beispielsweise in der Schiene des Bussystems 300 verläuft koppelbar ist. Das Hauptenergieversorgungskabel ist mit den einzelnen Sensoren 228, 36 in der Prozessiereinheit 10 oder der Adapterplatte 200 verbunden. So kann der Benutzer in wenigen Schritten, die Sensoren 228, 36 mit Energie versorgen.

Alternativ weist die zumindest eine Prozessiereinheit 10 und die zumindest eine Adapterplatte 200 eine integrierte Stromversorgung auf. Hierzu sind in die zumindest eine Prozessiereinheit 10 und die zumindest eine Adapterplatte 200 jeweils zumindest ein Teilabschnitt einer Stromversorgungsleitung integriert. Die Teilabschnitte der Stromversorgungsleitung sind miteinander konnektierbar sobald die einzelnen Komponenten (Prozessiereinheiten, Adapterplatten) zusammengefügt werden. Die Stromversorgungsleitung wird an einem Punkt von einer externen Energiequelle gespeist. Die einzelnen Sensoren 228, 38 sind mit der Stromversorgungsleitung verbunden. Vorzugsweise umfasst eine der Abschlusshalterungen 34, welche die Prozessiereinheiten 10 und Adapterplatten 200 sandwichartig halten, ebenfalls einen Teilabschnitt der Stromversorgungsleitung und die externe Energiequelle ist mit dem Teilabschnitt der Stromversorgungsleitung in der Abschlusshalterung 34 verbunden.

Die Energieversorgung wurde oben exemplarisch nur für Sensoren 228, 36 des Prozessiersystems 100 beschrieben. Es wird jedoch darauf hingewiesen, dass auch alle weiteren Elemente des Prozessiersystems 100, welche Energie benötigen, entweder einen Akku aufweisen oder von einem der oben beschriebenen kabelgebundenen Energieversorgungsmöglichkeiten gespeist werden können. Hierzu wird beispielsweise auf die Aktuatoren der Umlenkelemente, die Pumpe 258 oder das zumindest eine Ventil zur Drucksteuerung in der Adapterplatte 200 verwiesen.

Um Steuerbefehle von dem externen Steuergerät 400 zu den einzelnen Komponenten, wie z.B. die Aktuatoren der Umlenkelemente, dem zumindest einen Ventil zur Drucksteuerung oder die Pumpe 258 in der Adapterplatte 200 zu übertragen, kann hierzu ebenfalls das Bussystem 300 genutzt werden. Hierzu ist zumindest eine Steuer-Busleitung in dem Bussystem 300 vorgesehen, welche die Übertragung von Daten von dem externen Steuergerät 400 zu dem zu steuernden Element ermöglicht. Jedes der zu steuernden Elemente kann hierzu direkt mit einer separaten oder gemeinsamen Steuer-Busleitung verknüpft sein. Alternativ kann von der entsprechenden Steuer-Busleitung eine Haupt-Datenleitung z.B. in die Adapterplatte 200 geführt werden, wobei sich die Haupt-Datenleitung in der Adapterplatte 200 aufteilt, um die einzelnen zu steuernden Komponenten zu konnektieren.

Alternativ zu einer kabelgebundenen Datenübertragung können auch die Steuersignale kabellos übertragen werden, wie dies bezüglich der Datenübertragung zwischen den Sensoren 228, 36 und dem externen Steuergerät 400 beschrieben wurde. Das externe Steuergerät 400 kann hierzu selbst einen Transponder aufweisen, der die Übertragung von Steuerbefehlen an entsprechende Komponenten in dem Prozessiersystem 100, welche eine Regelung des Fluidstroms 14 ermöglichen, gewährleistet.

Das erfindungsgemäße Prozessiersystem 100 bietet somit eine Vielzahl von Vorteilen. Zum einen befindet sich zumindest ein Sensor 228, 36 eingebettet in dem Prozessiersystem 100, so dass ein aufwändiges Anschließen, Kalibrieren und Sterilisieren der Sensoren 228, 36 entfällt. Zudem umfasst das Prozessiersystem 100 nur ein externes Steuergerät 400, über welches die Messdaten der Sensoren 228, 36 gesammelt werden und gleichzeitig dafür genutzt werden, um den Fluidstrom 14 zu regeln. Für den Benutzer gibt es somit nur eine Anlaufstelle, über die er das Prozessiersystem 100 überwachen und gleichzeitig regeln kann.

Weiterhin wird die Datenübertragung in für den Benutzer einfacher Weise bereitgestellt. Sowohl eine kabellose als auch eine kabelgebundene Datenübertragung zwischen dem externen Steuergerät 400 und den einzelnen Komponenten des Prozessiersystems 100, die mit dem externen Steuergerät 400 kommunizieren, kann in einfacher und schneller Weise bereitgestellt werden. Eine aufwändige Verkabelung wird vermieden. Somit wird ein kompaktes und leicht zu bedienendes Prozessiersystem 100 bereitgestellt.

### Bezugszeichenliste

- 10: Prozessiereinheit
- 11: Prozessiereinheitengruppe
- 12: Prozessiergehäuse
- 13: Erste Prozessiereinheitengruppe
- 14: Fluidstrom
- 15: Zweite Prozessiereinheitengruppe
- 16: Filtermedium
- 17: Filterträger
- 18: Filtratseite
- 19: Leerraum
- 20: Retentatseite
- 21: Schüttgut
- 22: Oberes Ende des Prozessiergehäuses
- 24: Zulaufkanal
- 26: Ablaufkanal
- 27: Zweiter Ablaufkanal
- 30: Erste Prozessiereinheit
- 32: Zweite Prozessiereinheit
- 34: Abschlusshalterung
- 35: Zuflusspumpe
- 36: Prozessiereinheiten-Sensor
- 38: Prozessiereinheiten-Transponder
- 40: Filterkerze
- 42: Erste Seite der Filterkerze
- 44: Zweite Seite der Filterkerze
- 46: Filterkerzen-Ventil

- 100: Prozessiersystem

- 200: Adapterplatte
- 202: Erste Zutrittsöffnung
- 204: Unteres Ende der Adapterplatte
- 206: Zweite Zutrittsöffnung
- 208: Oberes Ende der Adapterplatte
- 210: Erste Austrittsöffnung
- 212: Zweite Austrittsöffnung
- 214: Adapterkanal
- 216: Erster Kanalbereich
- 218: Zweiter Kanalbereich
- 220: Verbindungskanalbereich
- 222: Umlenkventil
- 224: Erstes Umlenkventil
- 226: Zweites Umlenkventil
- 228: Adapterplatten-Sensor
- 230: Zulaufplatte
- 234: Kanalvertiefung
- 236: Strömungssteg
- 238: Verbindungskanal
- 240: Hilfsabzweigung
- 242: Kanalelement
- 244: Hilfsein- bzw. ausgang
- 246: Mehrwegventil
- 248: Ventilrohr
- 250: Mantelfläche des Ventilrohrs
- 252: Ventilöffnung
- 254: Verlagerungsgriff
- 257: Umlenkaktuator
- 258: Pumpe
- 260: Kolben
- 262: Zylinder
- 264: Einlassventil
- 266: Auslassventil
- 268: Schlauch
- 270: Rotor
- 272: Oberseite des Rotors
- 274: Rolle
- 276: Zahnradmechanismus
- 278: Statischer Mischer

- 300: Bussystem
- 302: Kabel
- 400: Externes Steuergerät

- HR: Horizontale Richtung
- VR: Vertikale Richtung

## Patentansprüche

1. Modulares Prozessiersystem (100) für biopharmazeutische und/oder chemische Prozesse umfassend:
- zumindest eine Prozessiereinheit (10) zur Durchführung eines Filtrationsschritts oder eines Chromatographieschritts in einem biopharmazeutischen oder chemischen Prozess;
- zumindest eine Adapterplatte (200), welche fluidisch mit der Prozessiereinheit (10) unmittelbar oder mittelbar verbunden ist, wobei die Adapterplatte (200) zumindest einen Adapterkanal (214) aufweist, der von zumindest einem Fluidstrom (14) durchströmbar ist, welcher zu der Prozessiereinheit (10) strömt, wobei die Adapterplatte (200) ferner zumindest ein als Mehrwegventil ausgebildetes Umlenkelement und/oder eine Pumpe (258) und/oder zumindest ein Ventil aufweist;
- Abschlusshalterungen (34), zwischen welchen die zumindest eine Prozessiereinheit (10) und die zumindest eine Adapterplatte (200) sandwichartig gehalten werden; und
- ein externes Steuergerät (400);
wobei die Adapterplatte (200) derart ausgebildet ist, dass der Fluidstrom (14) zu der Prozessiereinheit (10) mit Hilfe des zumindest einen als Mehrwegventil ausgebildeten Umlenkelements in dem Adapterkanal (214) zumindest teilweise umlenkbar ist und/oder der Fluidstrom (14), bevorzugt dessen Druck, mit Hilfe des zumindest einen Ventils und/oder der Pumpe (258) in dem Adapterkanal (214) regelbar ist;
wobei in der Prozessiereinheit (10) und/oder in der Adapterplatte (200) jeweils zumindest ein Sensor (36, 228) eingebettet ist, um zumindest eine Eigenschaft des Fluidstroms (14) in der Prozessiereinheit (10) bzw. der Adapterplatte (200) zu detektieren;
wobei das zumindest eine als Mehrwegventil ausgebildete Umlenkelement und/oder die Pumpe (258) und/oder das zumindest eine Ventil mittels eines Aktuators (257) steuerbar sind; und
wobei das externe Steuergerät (400) derart mit dem zumindest einen Sensor (36, 228) gekoppelt ist, dass Messdaten des zumindest einen Sensors (36, 228) ausgelesen werden können und basierend auf den ausgelesenen Messdaten das externe Steuergerät (400) den Aktuator (257) derart steuert, dass der Fluidstrom (14) in der Prozessiereinheit (10) und/oder der Adapterplatte (200) von dem externen Steuergerät (400) zentral geregelt werden kann.

2. Modulares Prozessiersystem (100) nach Anspruch 1, umfassend zumindest eine erste und zweite Prozessiereinheit (30, 32), welche fluidisch miteinander verbunden sind,
wobei der zumindest eine Adapterkanal (214) der Adapterplatte (200) von zumindest einem Fluidstrom (14) durchströmbar ist, welcher von der ersten Prozessiereinheit (30) zu der zweiten Prozessiereinheit (32) strömt; und
wobei die Adapterplatte (200) derart ausgebildet ist, dass der Fluidstrom (14) zwischen der ersten Prozessiereinheit (30) und der zweiten Prozessiereinheit (32) mit Hilfe des zumindest einen als Mehrwegventil ausgebildeten Umlenkelements in dem Adapterkanal (214) zumindest teilweise umlenkbar ist und der Fluidstrom (14), bevorzugt dessen Druck, mit Hilfe des zumindest einen Ventils und/oder der Pumpe (258) in dem Adapterkanal (214) regelbar ist.

3. Modulares Prozessiersystem (100) nach einem der vorangehenden Ansprüche, wobei in der ersten und zweiten Prozessiereinheit (30, 32) und in der Adapterplatte (200) jeweils zumindest ein Sensor (36, 228) eingebettet ist, um zumindest eine Eigenschaft des Fluidstroms (14) in der ersten und zweiten Prozessiereinheit (30, 32) und der Adapterplatte (200) zu detektieren.

4. Modulares Prozessiersystem (100) nach Anspruch 3, wobei das externe Steuergerät (400) derart mit den Sensoren (36, 228) gekoppelt ist, dass Messdaten der Sensoren (36, 228) ausgelesen werden können und basierend auf den ausgelesenen Messdaten der Fluidstrom (14) in den Prozessiereinheiten (30, 32) und der Adapterplatte (200) von dem externen Steuergerät (400) zentral geregelt werden kann.

5. Modulares Prozessiersystem (10) nach einem der vorangehenden Ansprüche,
wobei die zumindest eine Prozessiereinheit (10) und/oder die Adapterplatte (200) jeweils zumindest einen Transponder (38) aufweisen, der dazu ausgelegt ist, Messdaten eines entsprechenden Sensors (36, 228) an das externe Steuergerät (400) zu übertragen; oder
wobei die Sensoren (36, 228) der zumindest einen Prozessiereinheit (10) und/oder der zumindest einen Adapterplatte (200) mit dem externen Steuergerät (400) über ein Bussystem (300) gekoppelt sind.

6. Modulares Prozessiersystem (100) nach einem der vorangehenden Ansprüche, wobei der Sensor (36, 228) ausgebildet ist, einen Druck, einen Volumenstrom, einen UV-Wert, einen pH-Wert, eine Trübung und/oder eine Viskosität des Fluidstroms (14) zu messen.

7. Modulares Prozessiersystem (100) nach einem der vorangehenden Ansprüche, wobei der zumindest eine Sensor (36, 228), das zumindest eine als Mehrwegventil ausgebildete Umlenkelement, das zumindest eine Ventil und/oder die Pumpe (258) jeweils einen Akku aufweisen.

8. Modulares Prozessiersystem (100) nach einem der vorangehenden Ansprüche, wobei der zumindest eine Sensor (36, 228), das zumindest eine als Mehrwegventil ausgebildete Umlenkelement, das zumindest eine Ventil und/oder die Pumpe (258) eine kabelgebundene Stromversorgung aufweisen.

9. Modulares Prozessiersystem (100) nach Anspruch 8, wobei das Prozessiersystem (100) eine zentrale Stromversorgung für den zumindest einen Sensor (36, 228), das zumindest eine als Mehrwegventil ausgebildete Umlenkelement, das zumindest eine Ventil und/oder die Pumpe (258) aufweist,
wobei die zumindest eine Prozessiereinheit (10) und die zumindest eine Adapterplatte (200) Teilabschnitte der Stromversorgung aufweisen, die wenn zusammengefügt, die zentrale Stromversorgung bilden.

10. Modulares Prozessiersystem (100) nach einem der vorangehenden Ansprüche, wobei der zumindest eine Sensor (36, 228), das zumindest eine als Mehrwegventil ausgebildete Umlenkelement, das zumindest eine Ventil und die Pumpe (258) als Einweg-Elemente ausgebildet sind.

11. Verfahren zum zentralen Regeln eines modularen Prozessiersystems (100) für biopharmazeutische und/oder chemische Prozesse, umfassend die Schritte:
- Bereitstellen zumindest einer Prozessiereinheit (10) zur Durchführung eines Filtrationsschritts oder Chromatographieschritts in einem biopharmazeutischen oder chemischen Prozess;
- Bereitstellen zumindest einer Adapterplatte (200), welche zumindest einen Adapterkanal (214) aufweist, der von zumindest einem Fluidstrom (14) durchströmbar ist, wobei die Adapterplatte (200) ferner zumindest ein als Mehrwegventil ausgebildetes Umlenkelement und/oder eine Pumpe (258) und/oder zumindest ein Ventil aufweist, wobei das zumindest eine als Mehrwegventil ausgebildete Umlenkelement und/oder die Pumpe (258) und/oder das zumindest eine Ventil mittels eines Aktuators (257) steuerbar sind;
- Bereitstellen eines externen Steuergeräts (400);
- Mittelbares oder unmittelbares Verbinden der Adapterplatte (200) mit der Prozessiereinheit (10), so dass der Fluidstrom (14) von der Adapterplatte (200) zu der Prozessiereinheit (10) strömen kann;
- Bereitstellen von Abschlusshalterungen (34) und Anordnen der zumindest einen Prozessiereinheit (10) und der zumindest einen Adapterplatte (200) zwischen den Abschlusshalterungen (34), so dass die zumindest eine Prozessiereinheit (10) und die zumindest eine Adapterplatte (200) sandwichartig zwischen den Abschlusshalterungen (34) gehalten werden;
- Detektieren zumindest einer Eigenschaft des Fluidstroms (14) in der Prozessiereinheit (10) und/oder der Adapterplatte (200) mittels zumindest eines Sensors (36, 228), der in der Prozessiereinheit (10) und/oder der Adapterplatte (200) eingebettet ist; und
- Koppeln des externen Steuergeräts (400) mit dem zumindest einen Sensor (36, 228), so dass Messdaten des zumindest einen Sensors (36, 228) ausgelesen werden können; und
- Koppeln des externen Steuergeräts (400) mit dem zumindest einen als Mehrwegventil ausgebildeten Umlenkelement und/oder der Pumpe (258) und/oder dem zumindest einen Ventil in der Adapterplatte (200), so dass basierend auf den ausgelesenen Messdaten das externe Steuergerät (400) den Aktuator derart steuert, dass der Fluidstrom (14) in der Prozessiereinheit (10) und/oder der Adapterplatte (200) von dem externen Steuergerät (400) zentral geregelt werden kann;
wobei mit Hilfe des zumindest einen als Mehrwegventil ausgebildeten Umlenkelements in dem Adapterkanal (214) der Fluidstrom (14) zumindest teilweise umlenkbar ist, und/oder
wobei der Fluidstrom (14), bevorzugt dessen Druck, mit Hilfe des zumindest einen Ventils und/oder der Pumpe (258) in dem Adapterkanal (214) regelbar ist.

12. Verfahren nach Anspruch 11, wobei das Prozessiersystem (100) zumindest eine erste und zweite Prozessiereinheit (30, 32) umfasst; und
wobei die erste und zweite Prozessiereinheit (30, 32) mittels der Adapterplatte (200) derart miteinander gekoppelt werden, dass der Fluidstrom (14) von der ersten Prozessiereinheit (30) zu der zweiten Prozessiereinheit (32) strömen kann.

13. Verfahren nach Anspruch 12, wobei in der ersten und zweiten Prozessiereinheit (30, 32) und in der Adapterplatte (200) jeweils zumindest ein Sensor (36, 228) eingebettet ist;
wobei die Sensoren (36, 228) zumindest eine Eigenschaft des Fluidstroms (14) in der ersten und zweiten Prozessiereinheit (30, 32) und der Adapterplatte (200) detektieren; und
wobei die Sensoren (36, 228) mit dem externen Steuergerät (400) gekoppelt werden, so dass Messdaten der Sensoren (36, 228) ausgelesen werden können und basierend auf den ausgelesenen Messdaten der Fluidstrom (14) in den Prozessiereinheiten (30, 32) und der Adapterplatte (200) von dem externen Steuergerät (400) zentral geregelt werden kann.

## Claims

1. Modular processing system (100) for biopharmaceutical and/or chemical processes, comprising:
- at least one processing unit (10) for performing a filtration step or a chromatography step in a biopharmaceutical or chemical process;
- at least one adapter plate (200) that can be directly or indirectly fluidically connected to the processing unit (10), wherein the adapter plate (200) has at least one adapter channel (214) through which at least one fluid flow (14) can flow, which flows to the processing unit (10), wherein the adapter plate (200) additionally has at least one deflection element, designed as a multiway valve, and/or a pump (258) and/or at least one valve;
- termination brackets (34), between which the at least one processing unit (10) and the at least one adapter plate (200) are held in the manner of a sandwich; and
- an external control device (400);
wherein the adapter plate (200) is designed in such a way that the fluid flow (14) to the processing unit (10) can be at least partially deflected with the aid of the at least one deflection element, designed as a multiway valve, in the adapter channel (214), and/or the fluid flow (14), preferably the pressure thereof, can be regulated with the aid of the at least one valve and/or the pump (258) in the adapter channel (214);
wherein in each case at least one sensor (36, 228) is embedded in the processing unit (10) and/or in the adapter plate (200) in order to detect at least one property of the fluid flow (14) in the processing unit (10) and/or the adapter plate (200);
wherein the deflection element, designed as a multiway valve, and/or the pump (258) and/or the at least one valve are controllable by means of an actuator (257); and
wherein the external control device (400) is coupled to the at least one sensor (36, 228) in such a way that measurement data of the at least one sensor (36, 228) can be read out, and on the basis of the read-out measurement data the external control device (400) controls the actuator (257) in such a way that the fluid flow (14) in the processing unit (10) and/or the adapter plate (200) can be centrally regulated by the external control device (400).

2. Modular processing system (100) according to claim 1, comprising at least a first and a second processing unit (30, 32), which are fluidically connected to each other,
wherein at least one fluid flow (14) that flows from the first processing unit (30) to the second processing unit (32) can flow through the at least one adapter channel (214) of the adapter plate (200); and
wherein the adapter plate (200) is designed in such a way that the fluid flow (14) between the first processing unit (30) and the second processing unit (32) can be at least partially deflected with the aid of the at least one deflection element, designed as a multiway valve, in the adapter channel (214), and the fluid flow (14), preferably the pressure thereof, can be regulated with the aid of the at least one valve and/or the pump (258) in the adapter channel (214).

3. Modular processing system (100) according to any one of the preceding claims, wherein in each case at least one sensor (36, 228) is embedded in the first and the second processing unit (30, 32) and in the adapter plate (200) in order to detect at least one property of the fluid flow (14) in the first and the second processing unit (30, 32) and in the adapter plate (200) .

4. Modular processing system (100) according to claim 3, wherein the external control device (400) is coupled to the sensors (36, 228) in such a way that measurement data of the sensors (36, 228) can be read out, and on the basis of the read-out measurement data the fluid flow (14) in the processing units (30, 32) and the adapter plate (200) can be centrally regulated by the external control device (400).

5. Modular processing system (10) according to any one of the preceding claims,
wherein the at least one processing unit (10) and/or the adapter plate (200) each have at least one transponder (38) which is designed to transmit measurement data of a corresponding sensor (36, 228) to the external control device (400); or
wherein the sensors (36, 228) of the at least one processing unit (10) and/or the at least one adapter plate (200) are coupled to the external control device (400) via a bus system (300).

6. Modular processing system (100) according to any one of the preceding claims, wherein the sensor (36, 228) is designed to measure a pressure, a volumetric flow, a UV value, a pH value, a turbidity and/or a viscosity of the fluid flow (14).

7. Modular processing system (100) according to any one of the preceding claims, wherein the at least one sensor (36, 228), the at least one deflection element designed as a multiway valve, the at least one valve and/or the pump (258) each have a rechargeable battery.

8. Modular processing system (100) according to any one of the preceding claims, wherein the at least one sensor (36, 228), the at least one deflection element, designed as a multiway valve, the at least one valve and/or the pump (258) have a cable-based power supply.

9. Modular processing system (100) according to claim 8, wherein the processing system (100) has a central power supply for the at least one sensor (36, 228), the at least one deflection element, designed as a multiway valve, the at least one valve and/or the pump (258),
wherein the at least one processing unit (10) and the at least one adapter plate (200) have sub-sections of the power supply, which form the central power supply when assembled.

10. Modular processing system (100) according to any one of the preceding claims, wherein the at least one sensor (36, 228), the at least one deflection element, designed as a multiway valve, the at least one valve and the pump (258) are designed as single-use elements.

11. Method for centrally regulating a modular processing system (100) for biopharmaceutical and/or chemical processes, comprising the steps:
- providing at least one processing unit (10) for performing a filtration step or a chromatography step in a biopharmaceutical or chemical process;
- providing at least one adapter plate (200) which has at least one adapter channel (214) through which at least one fluid flow (14) can flow, wherein the adapter plate (200) additionally has at least one deflection element, designed as a multiway valve, and/or a pump (258) and/or at least one valve, wherein the at least one deflection element, designed as a multiway valve, and/or the pump (258) and/or the at least one valve are controllable by means of an actuator (257);
- providing an external control device (400);
- directly or indirectly connecting the adapter plate (200) to the processing unit (10) such that the fluid flow (14) can flow from the adapter plate (200) to the processing unit (10);
- providing termination brackets (34) and arranging the at least one processing unit (10) and the at least one adapter plate (200) between the termination brackets (34) such that the at least one processing unit (10) and the at least one adapter plate (200) are held between the termination brackets (34) in the manner of a sandwich;
- detecting at least one property of the fluid flow (14) in the processing unit (10) and/or the adapter plate (200) by means of at least one sensor (36, 228) which is embedded in the processing unit (10) and/or the adapter plate (200); and
- coupling the external control device (400) to the at least one sensor (36, 228) such that measurement data of the at least one sensor (36, 228) can be read out; and
- coupling the external control device (400) to the at least one deflection element, designed as a multiway valve, and/or the pump (258) and/or the at least one valve in the adapter plate (200) such that on the basis of the read-out measurement data the external control device (400) controls the actuator in such a way that the fluid flow (14) in the processing unit (10) and/or the adapter plate (200) can be centrally regulated by the external control device (400),
wherein the fluid flow (14) can be at least partially deflected with the aid of the at least one deflection element, designed as a multiway valve, in the adapter channel (214), and/or
wherein the fluid flow (14), preferably the pressure thereof, can be regulated with the aid of the at least one valve and/or the pump (258) in the adapter channel (214).

12. Method according to claim 11, wherein the processing system (100) comprises at least a first and a second processing unit (30, 32); and
wherein the first and the second processing unit (30, 32) are coupled to each other by means of the adapter plate (200) such that the fluid flow (14) can flow from the first processing unit (30) to the second processing unit (32).

13. Method according to claim 12, wherein in each case at least one sensor (36, 228) is embedded in the first and the second processing unit (30, 32) and in the adapter plate (200);
wherein the sensors (36, 228) detect at least one property of the fluid flow (14) in the first and the second processing unit (30, 32) and in the adapter plate (200); and
wherein the sensors (36, 228) are coupled to the external control device (400) such that measurement data of the sensors (36, 228) can be read out, and on the basis of the read-out measurement data the fluid flow (14) in the processing units (30, 32) and in the adapter plate (200) can be centrally regulated by the external control device (400).

## Revendications

1. Système de traitement modulaire (100) pour des processus biopharmaceutiques et/ou chimiques comprenant :
- au moins une unité de traitement (10) pour la mise en œuvre d'une étape de filtration ou d'une étape de chromatographie dans un processus biopharmaceutique ou chimique ;
- au moins une plaque d'adaptation (200), laquelle est reliée fluidiquement à l'unité de traitement (10) directement ou indirectement, dans lequel la plaque d'adaptation (200) présente au moins un canal d'adaptation (214), qui peut être traversé par au moins un courant de fluide (14), lequel s'écoule vers l'unité de traitement (10), dans lequel la plaque d'adaptation (200) présente en outre au moins un élément de déviation réalisé sous la forme d'une soupape multivoies et/ou une pompe (258) et/ou au moins une soupape ;
- des éléments de retenue terminaux (34), entre lesquels la au moins une unité de traitement (10) et la au moins une plaque d'adaptation (200) sont retenues en sandwich ; et
- un appareil de commande externe (400) ;
dans lequel la plaque d'adaptation (200) est réalisée de telle sorte que le courant de fluide (14) vers l'unité de traitement (10) peut être dévié au moins en partie dans le canal d'adaptation (214) à l'aide du au moins un élément de déviation réalisé sous la forme d'une soupape multivoies et/ou le courant de fluide (14), de préférence la pression de celui-ci, peut être régulé à l'aide de la au moins une soupape et/ou de la pompe (258) dans le canal d'adaptation (214) ;
dans lequel respectivement au moins un capteur (36, 228) est intégré dans l'unité de traitement (10) et/ou dans la plaque d'adaptation (200), afin de détecter au moins une propriété du courant de fluide (14) dans l'unité de traitement (10) ou la plaque d'adaptation (200) ;
dans lequel le au moins un élément de déviation réalisé sous la forme d'une soupape multivoies et/ou la pompe (258) et/ou la au moins une soupape peuvent être commandés au moyen d'un actionneur (257) ; et
dans lequel l'appareil de commande externe (400) est couplé au au moins un capteur (36, 228), de telle sorte que les données de mesure du au moins un capteur (36, 228) peuvent être lues et sur la base des données de mesure lues l'appareil de commande externe (400) commande l'actionneur (257) de telle sorte que le courant de fluide (14) dans l'unité de traitement (10) et/ou la plaque d'adaptation (200) peut être régulé de manière centrale par l'appareil de commande externe (400).

2. Système de traitement modulaire (100) selon la revendication 1, comprenant au moins une première et deuxième unité de traitement (30, 32), lesquelles sont reliées fluidiquement l'une à l'autre,
dans lequel le au moins un canal d'adaptation (214) de la plaque d'adaptation (200) peut être traversé par au moins un courant de fluide (14), lequel s'écoule à partir de la première unité de traitement (30) vers la deuxième unité de traitement (32) ; et
dans lequel la plaque d'adaptation (200) est réalisée de telle sorte que le courant de fluide (14) entre la première unité de traitement (30) et la deuxième unité de traitement (32) peut être dévié au moins en partie à l'aide du au moins un élément de déviation réalisé sous la forme d'une soupape multivoies dans le canal d'adaptation (214) et le courant de fluide (14), de préférence la pression de celui-ci, peut être régulé à l'aide de la au moins une soupape et/ou de la pompe (258) dans le canal d'adaptation (214).

3. Système de traitement modulaire (100) selon l'une quelconque des revendications précédentes, dans lequel respectivement au moins un capteur (36, 228) est intégré dans la première et deuxième unité de traitement (30, 32) et dans la plaque d'adaptation (200), afin de détecter au moins une propriété du courant de fluide (14) dans la première et deuxième unité de traitement (30, 32) et la plaque d'adaptation (200).

4. Système de traitement modulaire (100) selon la revendication 3, dans lequel l'appareil de commande externe (400) est couplé aux capteurs (36, 228) de telle sorte que les données de mesure des capteurs (36, 228) peuvent être lues et sur la base des données de mesure lues le courant de fluide (14) dans les unités de traitement (30, 32) et la plaque d'adaptation (200) peut être régulé de manière centrale par l'appareil de commande externe (400).

5. Système de traitement modulaire (10) selon l'une quelconque des revendications précédentes,
dans lequel la au moins une unité de traitement (10) et/ou la plaque d'adaptation (200) présentent respectivement au moins un transpondeur (38), qui est conçu pour transmettre les données de mesure d'un capteur (36, 228) correspondant à l'appareil de commande externe (400) ; ou
dans lequel les capteurs (36, 228) de la au moins une unité de traitement (10) et/ou de la au moins une plaque d'adaptation (200) sont couplés à l'appareil de commande externe (400) par l'intermédiaire d'un système de bus (300).

6. Système de traitement modulaire (100) selon l'une quelconque des revendications précédentes, dans lequel le capteur (36, 228) est réalisé pour mesurer une pression, un débit volumique, une valeur d'UV, une valeur de pH, un voile et/ou une viscosité du courant de fluide (14).

7. Système de traitement modulaire (100) selon l'une quelconque des revendications précédentes, dans lequel le au moins un capteur (36, 228), le au moins un élément de déviation réalisé sous la forme d'une soupape multivoies, la au moins une soupape et/ou la pompe (258) présentent respectivement une batterie rechargeable.

8. Système de traitement modulaire (100) selon l'une quelconque des revendications précédentes, dans lequel le au moins un capteur (36, 228), le au moins un élément de déviation réalisé sous la forme d'une soupape multivoies, la au moins une soupape et/ou la pompe (258) présentent une alimentation électrique filaire.

9. Système de traitement modulaire (100) selon la revendication 8, dans lequel le système de traitement (100) présente une alimentation électrique centrale pour le au moins un capteur (36, 228), le au moins un élément de déviation réalisé sous la forme d'une soupape multivoies, la au moins une soupape et/ou la pompe (258),
dans lequel la au moins une unité de traitement (10) et la au moins une plaque d'adaptation (200) présentent des sections partielles de l'alimentation électrique qui, lorsqu'elles sont réunies, forment l'alimentation électrique centrale.

10. Système de traitement modulaire (100) selon l'une quelconque des revendications précédentes, dans lequel le au moins un capteur (36, 228), le au moins un élément de déviation réalisé sous la forme d'une soupape multivoies, la au moins une soupape et la pompe (258) sont réalisés sous la forme d'éléments à usage unique.

11. Procédé pour la régulation centrale d'un système de traitement modulaire (100) pour des processus biopharmaceutiques et/ou chimiques, comprenant les étapes de :
- fourniture d'au moins une unité de traitement (10) pour la mise en œuvre d'une étape de filtration ou étape de chromatographie dans un processus biopharmaceutique ou chimique ;
- fourniture d'au moins une plaque d'adaptation (200), laquelle présente au moins un canal d'adaptation (214), qui peut être traversé par au moins un courant de fluide (14), dans lequel la plaque d'adaptation (200) présente en outre au moins un élément de déviation réalisé sous la forme d'une soupape multivoies et/ou une pompe (258) et/ou au moins une soupape, dans lequel le au moins un élément de déviation réalisé sous la forme d'une soupape multivoies et/ou la pompe (258) et/ou la au moins une soupape peuvent être commandés au moyen d'un actionneur (257) ;
- fourniture d'un appareil de commande externe (400) ;
- liaison directe ou indirecte de la plaque d'adaptation (200) à l'unité de traitement (10), de sorte que le courant de fluide (14) peut circuler de la plaque d'adaptation (200) à l'unité de traitement (10) ;
- fourniture d'éléments de retenue terminaux (34) et disposition de la au moins une unité de traitement (10) et la au moins une plaque d'adaptation (200) entre les éléments de retenue terminaux (34), de sorte que la au moins une unité de traitement (10) et la au moins une plaque d'adaptation (200) sont retenues en sandwich entre les éléments de retenue terminaux (34) ;
- détection d'au moins une propriété du courant de fluide (14) dans l'unité de traitement (10) et/ou la plaque d'adaptation (200) au moyen d'au moins un capteur (36, 228), qui est intégré dans l'unité de traitement (10) et/ou la plaque d'adaptation (200) ; et
- couplage de l'appareil de commande externe (400) au au moins un capteur (36, 228), de sorte que les données de mesure du au moins un capteur (36, 228) peuvent être lues ; et
- couplage de l'appareil de commande externe (400) au au moins un élément de déviation réalisé sous la forme d'une soupape multivoies et/ou la pompe (258) et/ou la au moins une soupape dans la plaque d'adaptation (200), de sorte que l'appareil de commande externe (400) commande l'actionneur sur la base des données de mesure lues, de telle sorte que le courant de fluide (14) dans l'unité de traitement (10) et/ou la plaque d'adaptation (200) peut être régulé de manière centrale par l'appareil de commande externe (400) ;
dans lequel à l'aide du au moins un élément de déviation réalisé sous la forme d'une soupape multivoies le courant de fluide (14) peut être dévié au moins en partie dans le canal d'adaptation (214), et/ou
dans lequel le courant de fluide (14), de préférence la pression de celui-ci, peut être régulé à l'aide de la au moins une soupape et/ou de la pompe (258) dans le canal d'adaptation (214).

12. Procédé selon la revendication 11, dans lequel le système de traitement (100) comprend au moins une première et deuxième unité de traitement (30, 32) ; et
dans lequel la première et deuxième unité de traitement (30, 32) sont couplées l'une à l'autre au moyen de la plaque d'adaptation (200), de telle sorte que le courant de fluide (14) peut circuler de la première unité de traitement (30) à la deuxième unité de traitement (32).

13. Procédé selon la revendication 12, dans lequel respectivement au moins un capteur (36, 228) est intégré dans la première et deuxième unité de traitement (30, 32) et dans la plaque d'adaptation (200) ;
dans lequel les capteurs (36, 228) détectent au moins une propriété du courant de fluide (14) dans la première et deuxième unité de traitement (30, 32) et la plaque d'adaptation (200) ; et
dans lequel les capteurs (36, 228) sont couplés à l'appareil de commande externe (400), de sorte que les données de mesure des capteurs (36, 228) peuvent être lues et le courant de fluide (14) dans les unités de traitement (30, 32) et la plaque d'adaptation (200) peut être régulé de manière centrale par l'appareil de commande externe (400) sur la base des données de mesure lues.
